(19) Europäisches Patentamt
European Patent Office
Office européen des brevets

(11) Veröffentlichungsnummer: **0 045 451**
**A2**

(12) # EUROPÄISCHE PATENTANMELDUNG

(21) Anmeldenummer: **81105834.6**

(22) Anmeldetag: **23.07.81**

(51) Int. Cl.$^3$: **C 07 D 243/24**
A 61 K 31/55, C 07 D 403/04
C 07 D 243/26, C 07 D 243/28

(30) Priorität: **31.07.80 CH 5842/80**

(43) Veröffentlichungstag der Anmeldung:
**10.02.82 Patentblatt 82/6**

(84) Benannte Vertragsstaaten:
**AT BE CH DE FR GB IT LI LU NL SE**

(71) Anmelder: **F. HOFFMANN-LA ROCHE & CO. Aktiengesellschaft**

**CH-4002 Basel(CH)**

(72) Erfinder: **Branca, Quirico, Dr.**
**Lenzgasse 2**
**CH-4056 Basel(CH)**

(72) Erfinder: **Fischli, Albert Eduard, Prof. Dr.**
**Am Ausserberg 20**
**CH-4125 Riehen(CH)**

(72) Erfinder: **Szente, André, Dr.**
**Baselstrasse 70**
**CH-4125 Riehen(CH)**

(74) Vertreter: **Lederer, Franz, Dr. et al,**
**Patentanwälte Dr. Lederer Franz Meyer-Roxlau Reiner F.**
**Lucile-Grahn-Strasse 22**
**D-8000 München 80(DE)**

(54) **Benzodiazepin-Derivate, Zwischenprodukte und Verfahren zu ihrer Herstellung sowie diese enthaltende Arzneimittel.**

(57) Die neuen Benzodiazepin-Derivate der allgemeinen Formel

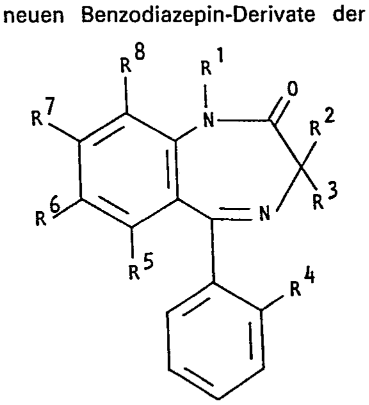

1

worin $R^1$ niederes Alkyl, niederes Hydroxyalkyl oder niederes Dialkylaminoalkyl, $R^2$ und $R^3$ je niederes Alkyl, $R^4$ Halogen, $R^5$, $R^7$ und $R^8$ je Wasserstoff oder Halogen und $R^6$ Nitro, Amino, niederes Alkylamino, niederes Dialkylamino oder einen Rest der Formel $H_2N\text{-}C(CH_3)_2\text{-}CO\text{-}NH\text{-}$, $R^9R^{10}N\text{-}CO\text{-}NH\text{-}$ oder

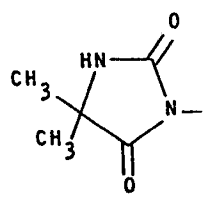

bedeuten und entweder $R^9$ Wasserstoff oder niederes Alkyl und $R^{10}$ niederes Alkyl oder niederes Hydroxyalkyl bedeuten oder $R^9$ und $R^{10}$ zusammen mit dem Stickstoffatom einen 3- bis 7-gliedrigen Heterocyclus bedeuten, der - falls er mindestens 5-gliedrig ist - ein Sauerstoff- oder Schwefelatom oder einen Rest der Formel >N-R als Ringglied enthalten kann, wobei R Wasserstoff oder niederes Alkyl bedeutet,

und ihre pharmazeutisch annehmbaren Säureadditionssalze besitzen ausgeprägte aldosteronantagonistische Eigenschaften und eignen sich demnach bei der Bekämpfung bzw. Verhütung von Herzversagen, von hepatitischer Aszites, von primärem Aldosteronismus und von essentieller Hypertonie. Diese Verbindungen können ausgehend von zum Teil neuen Zwischenprodukten nach verschiedenen Verfahren hergestellt und in galenische Darreichungsformen gebracht werden.

EP 0 045 451 A2

Croydon Printing Company Ltd.

0045451

23. Juli 1981

F.Hoffmann-La Roche & Co. Aktiengesellschaft, Basel, Schweiz

RAN 4008/317

# Benzodiazepin-Derivate, Zwischenprodukte und Verfahren zu ihrer Herstellung sowie diese enthaltende Arzneimittel

Die vorliegende Erfindung betrifft Benzodiazepin-Derivate. Im speziellen betrifft sie 3,3-Dialkylbenzodiazepin-Derivate der allgemeinen Formel

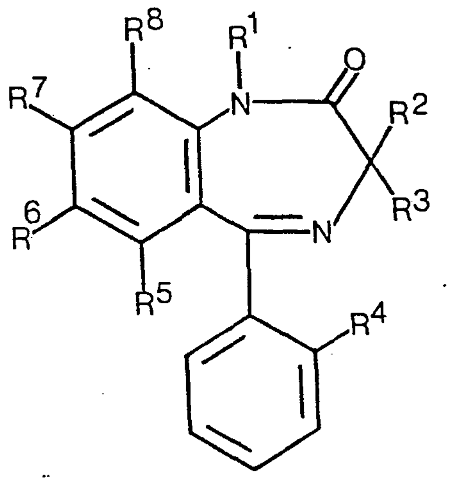

I

worin $R^1$ niederes Alkyl, niederes Hydroxyalkyl oder niederes Dialkylaminoalkyl, $R^2$ und $R^3$ je niederes Alkyl, $R^4$ Halogen, $R^5$, $R^7$ und $R^8$ je Wasserstoff oder Halogen und $R^6$ Nitro, Amino, niederes Alkylamino, niederes Dialkylamino oder einen Rest der Formel

Nt/ 23.4.1981

$H_2N-C(CH_3)_2-CO-NH-$, $R^9R^{10}N-CO-NH-$ oder 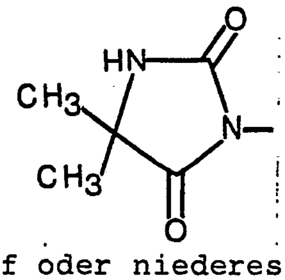

bedeuten und entweder $R^9$ Wasserstoff oder niederes Alkyl und $R^{10}$ niederes Alkyl oder niederes Hydroxyalkyl bedeuten oder $R^9$ und $R^{10}$ zusammen mit dem Stickstoffatom einen 3- bis 7-gliedrigen Heterocyclus bedeuten, der - falls er mindestens 5-gliedrig ist - ein Sauerstoff- oder Schwefelatom oder einen Rest der Formel $>N-R$ als Ringglied enthalten kann, wobei R Wasserstoff oder niederes Alkyl bedeutet,

und pharmazeutisch annehmbare Säureadditionssalze davon.

Diese Verbindungen sind neu und zeichnen sich durch wertvolle pharmakodynamische Eigenschaften aus.

Gegenstand der vorliegenden Erfindung sind 3,3-Dialkylbenzodiazepin-Derivate der obigen allgemeinen Formel I und pharmazeutisch annehmbare Säureadditionssalze davon als solche und als pharmazeutische Wirkstoffe, die Herstellung dieser Verbindungen und Zwischenprodukte für die Herstellung dieser Verbindungen, ferner Arzneimittel, enthaltend eine oder mehrere Verbindungen der allgemeinen Formel I oder pharmazeutisch annehmbare Säureadditionssalze davon und die Herstellung solcher Arzneimittel sowie die Verwendung von 3,3-Dialkylbenzodiazepin-Derivaten der allgemeinen Formel I und von pharmazeutisch annehmbaren Säureadditionssalzen davon bei der Bekämpfung bzw. Verhütung von Krankheiten.

Der Ausdruck "niederes Alkyl", für sich allein genommen oder in Kombinationen, wie "niederes Hydroxyalkyl", "niederes Dialkylaminoalkyl", "niederes Alkylamino", "niederes Dialkylamino" und dergleichen, bezeichnet geradkettige oder verzweigte gesättigte Kohlenwasserstoffreste mit höchstens 7, vorzugsweise höchstens 4 Kohlenstoffatomen, wie Methyl, Aethyl, n-Propyl, Isopropyl, n-Butyl, t-Butyl usw. Der Ausdruck "niederes Alkylen", wie er in der

vorliegenden Beschreibung verwendet wird, bezeichnet zweiwertige, gesättigte Kohlenwasserstoffreste mit höchstens 7, vorzugsweise höchstens 4 Kohlenstoffatomen, welche geradkettig oder verzweigt sein können, wie Methylen, Aethylen, 1,2-Propylen, Aethyliden und dergleichen. Der Ausdruck "niederes Hydroxyalkyl", umfasst Reste wie 2-Hydroxyäthyl, 3-Hydroxy-2-propyl und dergleichen. Der Ausdruck "niederes Dialkylaminoalkyl" umfasst Reste, wie 2-Diäthylaminoäthyl, 2-Dimethylaminoäthyl, 3-(Methyl-äthylamino)-butyl und dergleichen. Der Ausdruck "niederes Alkylamino" bedeutet beispielsweise Methylamino, Aethylamino, Isopropylamino, n-Butylamino und dergleichen. Der Ausdruck "niederes Dialkylamino" bezeichnet Reste, wie Dimethylamino, Diäthylamino, Diisopropylamino und dergleichen. Der Ausdruck "Halogen" bedeutet Fluor, Chlor, Brom oder Jod.

Der Ausdruck "Heterocyclus", wie er oben näher umschrieben wird, bedeutet heterocyclische Reste, wie Aziridin-1-yl, Pyrrolidin-1-yl, 4-Methyl-piperazin-1-yl, Morpholin-4-yl und dergleichen.

Unter den Verbindungen die von der allgemeinen Formel I umfasst werden, sind diejenigen bevorzugt, worin $R^1$ Methyl bedeutet. $R^2$ und $R^3$ bedeuten vorzugsweise beide Methyl. $R^4$ bedeutet vorzugsweise Fluor oder Chlor. Die bevorzugte Bedeutungsmöglichkeit von $R^5$ ist Wasserstoff, Chlor oder Brom. $R^7$ bedeutet vorzugsweise Wasserstoff, Chlor oder Brom. Für $R^8$ sind die Bedeutungsmöglichkeiten Wasserstoff und Chlor bevorzugt.

Besonders bevorzugte von der allgemeinen Formel I umfasste Verbindungen im Rahmen der vorliegenden Erfindung sind solche Verbindungen, worin $R^1$, $R^2$ und $R^3$ alle Methyl bedeuten.

Ganz besonders bevorzugte Verbindungen der allgemeinen Formel I sind:

7-Amino-9-chlor-5-(o-fluorphenyl)-1,3-dihydro-1,3,3-trimethyl-2H-1,4-benzodiazepin-2-on;

7-Amino-6,8-dibrom-5-(o-chlorphenyl)-1,3-dihydro-1,3,3-trimethyl-2H-1,4-benzodiazepin-2-on;

7-Amino-6,8-dibrom-5-(o-fluorphenyl)-1,3-dihydro-1,3,3-trimethyl-2H-1,4-benzodiazepin-2-on und

7-Amino-6,8-dichlor-5-(o-chlorphenyl)-1,3-dihydro-1,3,3-trimethyl-2H-1,4-benzodiazepin-2-on.

Weitere bevorzugte Verbindungen der allgemeinen Formel I sind:

1-[6-Brom-5-(o-chlorphenyl)-2,3-dihydro-1,3,3-trimethyl-2-oxo-1H-1,4-benzodiazepin-7-yl]-3-(2-hydroxyäthyl)harnstoff;

7-Amino-9-chlor-5-(o-chlorphenyl)-1,3-dihydro-1,3,3-trimethyl-2H-1,4-benzodiazepin-2-on;

1-[5-(o-chlorphenyl)-2,3-dihydro-1,3,3-trimethyl-2-oxo-1H-1,4-benzodiazepin-7-yl]-3-(2-hydroxyäthyl)harnstoff;

7-Amino-6,8,9-trichlor-1,3-dihydro-5-(o-fluorphenyl)-1,3,3-trimethyl-2H-1,4-benzodiazepin-2-on

7-Amino-8,9-dichlor-5-(o-fluorphenyl)-1,3-dihydro-1,3,3-trimethyl-2H-1,4-benzodiazepin-2-on;

7-Amino-6-chlor-5-(o-fluorphenyl)-1,3-dihydro-1,3,3-trimethyl-2H-1,4-benzodiazepin-2-on und

2-Amino-N-[5-(o-fluorphenyl)-2,3-dihydro-1,3,3-trimethyl-2-oxo-1H-1,4-benzodiazepin-7-yl]-2-methylpropion-amid.

Die neuen 3,3-Dialkylbenzodiazepin-Derivate der allgemeinen Formel I und ihre pharmazèutisch annehmbaren Säureadditionssalze können erfindungsgemäss hergestellt werden, indem man

a) eine Verbindung der allgemeinen Formel

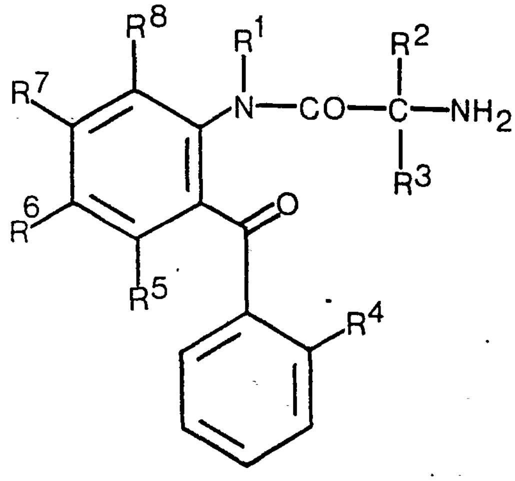

II

worin $R^1$, $R^2$, $R^3$, $R^4$, $R^5$, $R^6$, $R^7$ und $R^8$ obige Bedeutung besitzen,

cyclisiert, oder

b) aus einer Verbindung der allgemeinen Formel

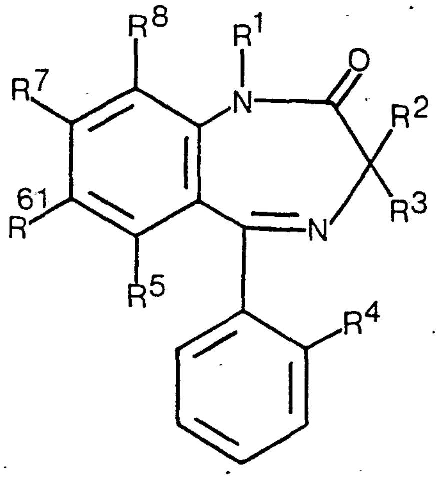

III

worin $R^1$, $R^2$, $R^3$, $R^4$, $R^5$, $R^7$ und $R^8$ obige Bedeutung besitzen und $R^{61}$ geschütztes niederes Alkylamino oder einen Rest der Formel ZZ'N-, Z-NH-C($CH_3$)$_2$-CO-NH- oder $R^{91}R^{101}$N-CO-NH- bedeuten und entweder $R^{91}$ Wasserstoff oder niederes Alkyl und $R^{101}$ einen Rest der Formel -A-O-Z bedeuten oder $R^{91}$ eine Schutzgruppe und $R^{101}$ niederes Alkyl oder einen Rest der Formel -A-O-Z bedeuten, oder $R^{91}$ und $R^{101}$ zusammen mit dem Stickstoffatom einen 5- bis 7-gliedrigen Heterocyclus bedeuten, der einen Rest der Formel >N-$R^{01}$ als Ringglied enthält, wobei $R^{01}$ eine Schutzgruppe bedeutet, und A niederes Alkylen und Z und Z' je eine Schutzgruppe bedeuten, wobei Z zusammen mit Z' eine Schutzgruppe bedeuten kann, die Schutzgruppe(n) abspaltet, oder

c) ein 3,3-Dialkylbenzodiazepin-Derivat der allgemeinen Formel

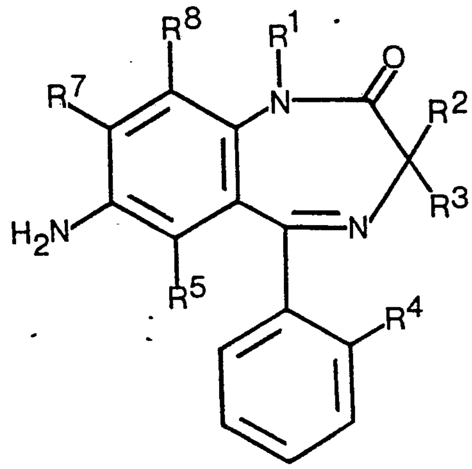

Ia

worin $R^1$, $R^2$, $R^3$, $R^4$, $R^5$, $R^7$ und $R^8$ obige Bedeutung besitzen,

mit einem Halogenid der allgemeinen Formel

$$R^{92}R^{102}N\text{-}CO\text{-}X \qquad IV$$

worin X Halogen bedeutet und entweder $R^{92}$ und $R^{102}$ je niederes Alkyl bedeuten oder $R^{92}$ und $R^{102}$ zusammen mit dem Stickstoffatom einen 3- bis 7-gliedrigen Heterocyclus bedeuten, der - falls er mindestens 5-gliedrig ist - ein Sauerstoff- oder Schwefelatom oder einen Rest der Formel $>N\text{-}R^{02}$ enthalten kann, wobei $R^{02}$ niederes Alkyl bedeutet,

umsetzt, oder

d) ein 3,3-Dialkylbenzodiazepin-Derivat der obigen allgemeinen Formel Ia mit einem Isocyanat der allgemeinen Formel

$$R^{93}\text{-}NCO \qquad V$$

worin $R^{93}$ niederes Alkyl bedeutet,

umsetzt, oder

e) eine Verbindung der allgemeinen Formel

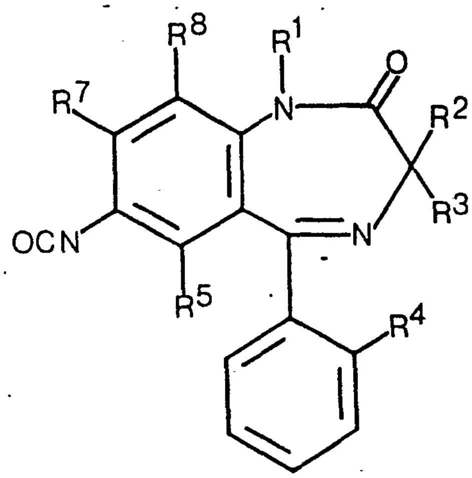

VI

worin $R^1$, $R^2$, $R^3$, $R^4$, $R^5$, $R^7$ und $R^8$ obige Bedeutung besitzen, mit einer Aminoverbindung der allgemeinen Formel

$R^9R^{10}NH$ VII

worin $R^9$ und $R^{10}$ obige Bedeutung besitzen, umsetzt, oder

f) eine Verbindung der allgemeinen Formel

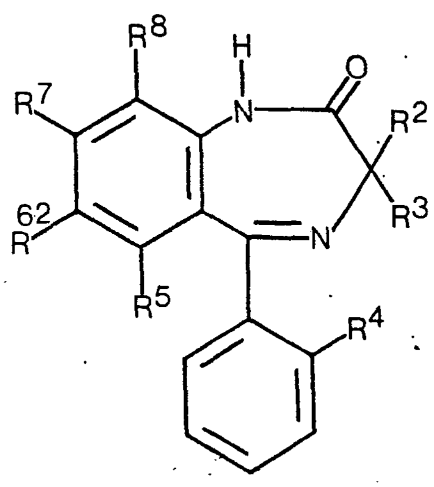

VIII

worin $R^2$, $R^3$, $R^4$, $R^5$, $R^7$ und $R^8$ obige Bedeutung besitzen und $R^{62}$ Nitro oder niederes Dialkylamino bedeutet,

mit einem den Rest $R^1$ liefernden Alkylierungsmittel behandelt, oder

g) ein 3,3-Dialkylbenzodiazepin-Derivat der allgemeinen Formel

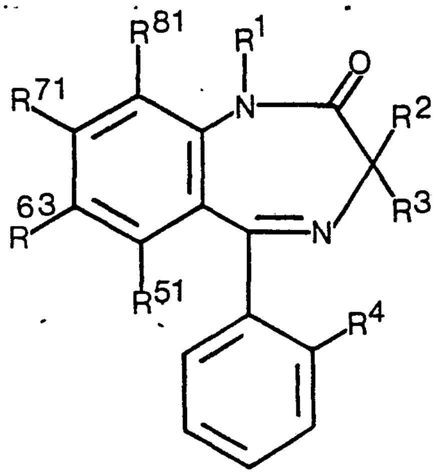

Ib

worin $R^1$, $R^2$, $R^3$ und $R^4$ obige Bedeutung besitzen, und entweder $R^{63}$ Nitro und $R^{51}$, $R^{71}$ und $R^{81}$ alle Wasserstoff bedeuten oder $R^{63}$ Amino oder niederes Alkylamino und $R^{81}$ Wasserstoff oder Halogen bedeuten, und einer der Reste $R^{51}$ und $R^{71}$ Wasserstoff und der andere Wasserstoff oder Halogen bedeutet,

halogeniert, oder

h) in einem 3,3-Dialkylbenzodiazepin-Derivat der obigen allgemeinen Formel Ia die primäre Aminogruppe in die Nitrogruppe überführt, oder

i) in einem 3,3-Dialkylbenzodiazepin-Derivat der allgemeinen Formel

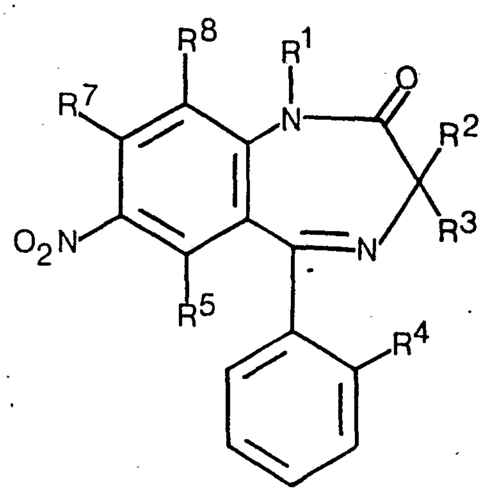

Ic

worin $R^1$, $R^2$, $R^3$, $R^4$, $R^5$, $R^7$ und $R^8$ obige Bedeutung besitzen,

die Nitrogruppe zur Aminogruppe reduziert, oder

j) in einem 3,3-Dialkylbenzodiazepin-Derivat der obigen allgemeinen Formel Ia die primäre Aminogruppe mono- oder dialkyliert, oder

k) in einem 3,3-Dialkylbenzodiazepin-Derivat der allgemeinen Formel

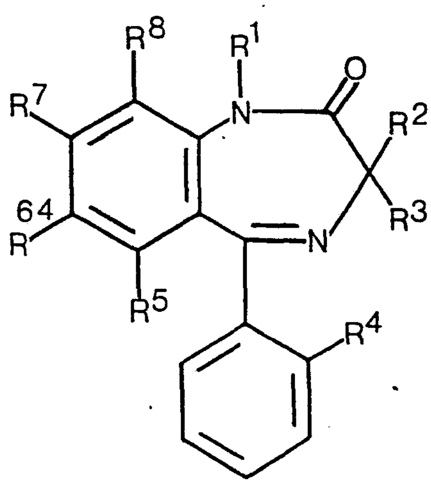

Id

worin $R^1$, $R^2$, $R^3$, $R^4$, $R^5$, $R^7$ und $R^8$ obige Bedeutung besitzen und $R^{64}$ niederes Alkylamino bedeutet,

die sekundäre Aminogruppe alkyliert, oder

l) ein 3,3-Dialkylbenzodiazepin-Derivat der obigen allgemeinen Formel Ia mit einem Dihalogenid der allgemeinen Formel

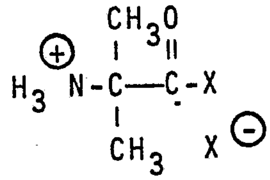

IX

worin X obige Bedeutung besitzt,

umsetzt, oder

m) ein 3,3-Dialkylbenzodiazepin-Derivat der allgemeinen Formel

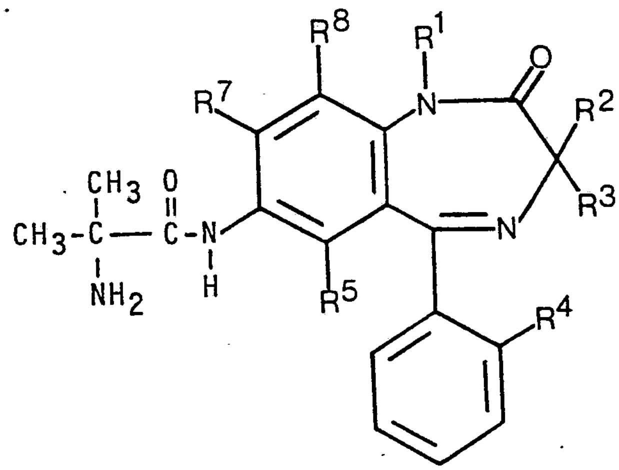

Ie

worin $R^1$, $R^2$, $R^3$, $R^4$, $R^5$, $R^7$ und $R^8$ obige Bedeutung besitzen,

mit Phosgen behandelt, oder

n) in einem 3,3-Dialkylbenzodiazepin-Derivat der allgemeinen Formel

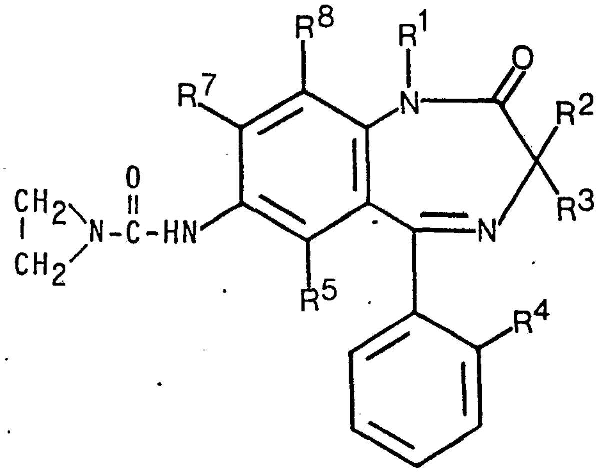

If

worin $R^1$, $R^2$, $R^3$, $R^4$, $R^5$, $R^7$ und $R^8$ obige Bedeutung besitzen,
den Aziridinring hydrolytisch öffnet, oder

o) ein 3,3-Dialkylbenzodiazepin-Derivat der allgemeinen Formel I in ein pharmazeutisch annehmbares Säureadditionssalz überführt.

Gemäss Verfahrensvariante a) kann ein 3,3-Dialkylbenzodiazepin-Derivat der allgemeinen Formel I hergestellt werden, indem man eine Verbindung der allgemeinen Formel II cyclisiert. Diese Ringschlussreaktion erfolgt ziemlich leicht und kann gegebenenfalls durch längeres Stehenlassen und/oder durch Anwendung von Wärme beschleunigt werden. Man kann in neutralem, alkalischem oder in saurem Milieu arbeiten. Die Cyclisierung erfolgt zweckmässigerweise in einem inerten organischen Lösungsmittel, z.B. in Kohlenwasserstoffen, wie Benzol, Toluol usw., in chlorierten Kohlenwasserstoffen, wie Chloroform, Methylenchlorid usw., in Aether, wie Dioxan, usw. Für die Cyclisierung der Verbindungen der allgemeinen FormelII eignen sich Temperaturen im Bereich zwischen Raumtemperatur und etwa 150°C, natürlich unter Berücksichtigung des eingesetzten Lösungsmittels. In einer bevorzugten Ausführungsform wendet man Bedingungen an, die ein azeotropes Entfernen des gebildeten Reaktionswassers ermöglichen.

Die Verbindungen der allgemeinen Formel II müssen nicht notwendigerweise in isoliertem Zustand eingesetzt werden, und in manchen Fällen ist dies auch nicht möglich. In der Regel erweist es sich als zweckmässig, die Verbindungen der allgemeinen Formel II ohne Isolierung aus dem Reaktionsgemisch, in dem sie hergestellt worden sind, direkt zu cyclisieren bzw. cyclisieren zu lassen.

Gemäss Verfahrensvariante b) kann ein 3,3-Dialkylbenzodiazepin-Derivat der allgemeinen Formel I hergestellt werden, indem man aus einem Benzodiazepin-Derivat der allgemeinen Formel III die Schutzgruppe bzw. die Schutzgruppen entfernt. Als Stickstoff-Schutzgruppen eignen sich für die Zwecke der vorliegenden Erfindung in erster Linie Acylgruppen, vorzugsweise leicht abspaltbare Alkoxycarbonyl- oder Aralkoxycarbonylgruppen, insbesondere die t-Butoxycarbonylgruppe, die Benzyloxycarbonylgruppe usw., ferner auch leicht abspaltbare Aralkylgruppen, wie die Benzylgruppe. Als Sauerstoff-Schutzgruppen eignen sich einerseits Acylgruppen oder Aralkylgruppen, wie sie soeben als Stickstoff-Schutzgruppen erwähnt worden sind, andererseits aber auch Acetal- und Ketal-Schutzgruppen, wie Tetrahydro-2-pyranyl, 2-Methoxy-2-propyl, Methoxymethyl, β-Methoxyäthoxy-methyl usw., leicht abspaltbare Alkylgruppen, wie t-Butyl usw., oder Alkanoylgruppen, wie Acetyl und dergleichen.

Die Entfernung der Schutzgruppe bzw. der Schutzgruppen aus den Benzodiazepin-Derivaten der allgemeinen Formel III erfolgt nach an sich bekannten Methoden, wobei natürlich die Natur der zu entfernenden Schutzgruppe bzw. Schutzgruppen für die Wahl der zur Anwendung gelangenden Methode bzw. Methoden in Betracht gezogen werden muss. Ebenfalls zu beachten ist natürlich, dass nur solche Methoden verwendet werden können, welche die Schutzgruppe bzw. Schutzgruppen selektiv entfernen, ohne dass andere

im Molekül vorhandene Strukturelemente in Mitleidenschaft gezogen werden.

Die weiter oben als Beispiele für in Betracht kommende Schutzgruppen erwähnten Reste können je nach ihrer Natur hydrogenolytisch und/oder hydrolytisch abgespalten werden. So können z.B. die Benzyloxycarbonylgruppe und die t-Butoxycarbonylgruppe unter selektiven sauren Bedingungen abgespalten werden, z.B. durch Behandeln mit einem Gemisch von Bromwasserstoff und Eisessig oder durch Behandeln mit Bortrifluorid oder Bortribromid in einem inerten organischen Lösungsmittel, wie Dichlormethan. Die t-Butoxycarbonylgruppe kann auch durch Behandeln mit Chlorwasserstoff in einem inerten organischen Lösungsmittel, wie Dioxan, Tetrahydrofuran oder dergleichen, oder durch Behandeln mit Trifluoressigsäure abgespalten werden. Die Tetrahydropyranylgruppe lässt sich unter milden sauren Bedingungen abspalten, beispielsweise durch Behandeln mit verdünnter wässriger Mineralsäure oder durch Umacetalisieren mit einem niederen Alkohol, wie z.B. Methanol oder Aethanol, in Gegenwart eines sauren Katalysators, wie z.B. Salzsäure, p-Toluolsulfonsäure, Pyridinium-p-toluolsulfonat oder dergleichen. Die t-Butylgruppe kann z.B. mittels Trifluoressigsäure abgespalten werden. Die Benzylgruppe kann durch katalytische Hydrierung, z.B. über Palladium/Kohle entfernt werden. Die Acetylgruppe kann unter milden alkalischen Bedingungen abgespalten werden, z.B. mit einer Lösung eines Natriumalkoholats im entsprechenden Alkohol (wie methanolisches Natriummethylat).

Bedeuten Z und Z' in Verbindungen der allgemeinen Formel III, worin $R^{61}$ einen Rest der Formel ZZ'N- bedeutet, eine einzige Schutzgruppe, so kommen in erster Linie cyclische Imide, beispielsweise Phthalimide, in Frage. Eine solche Schutzgruppe lässt sich, z.B. mit Hydrazin, leicht wieder entfernen.

Gemäss Verfahrensvariante c) kann ein 3,3-Dialkylbenzodiazepin-Derivat der allgemeinen Formel I hergestellt werden, indem man ein Benzodiazepin-Derivat der allgemeinen Formel Ia mit einem Halogenid der allgemeinen Formel IV umsetzt. Diese Reaktion erfolgt in Gegenwart eines säurebindenden Mittels, beispielsweise einer anorganischen Base, wie Kaliumcarbonat, Natriumcarbonat usw., oder einer organischen Base, z.B. einer tertiären Aminoverbindung, wie Triäthylamin, N-Aethyl-diisopropylamin, Chinuclidin und dergleichen.

Die Umsetzung der Verbindungen der Formeln Ia und IV erfolgt zweckmässigerweise bei Raumtemperatur oder unterhalb davon; sie kann ziemlich langsam verlaufen und dauert in der Regel mehrere Tage.

Gemäss Verfahrensvariante d) kann ein 3,3-Dialkylbenzodiazepin-Derivat der allgemeinen Formel I hergestellt werden, indem man ein Benzodiazepin-Derivat der allgemeinen Formel Ia mit einem Isocyanat der allgemeinen Formel V umsetzt. Diese Reaktion erfolgt zweckmässigerweise in einem unter den Reaktionsbedingungen inerten organischen Lösungsmittel, beispielsweise in einem halogenierten Kohlenwasserstoff, wie z.B. Methylenchlorid, Dichloräthan, Chloroform oder o-Dichlorbenzol, in einem Aether, wie z.B. Tetrahydrofuran, Dioxan, Dimethoxyäthan oder Diäthylenglykoldimethyläther, oder dergleichen. In manchen Fällen erweist es sich als günstig, die Reaktion in Gegenwart einer katalytisch wirkenden geringen Menge einer Base durchzuführen, beispielsweise in Gegenwart einer tertiären Aminoverbindung, wie Triäthylamin, N-Aethyl-diisopropylamin, Chinuclidin usw.. Die Temperatur ist für die Umsetzung der Verbindungen der Formeln Ia und V nicht kritisch, und man kann demnach bei Raumtemperatur, unterhalb oder oberhalb davon arbeiten, beispielsweise bei Rückflusstemperatur.

Gemäss Verfahrensvariante e) kann ein 3,3-Dialkylbenzodiazepin-Derivat der allgemeinen Formel I hergestellt

werden, indem man ein Benzodiazepin-Derivat der allgemeinen Formel VI mit einer Aminoverbindung der Formel VII umsetzt. Zweckmässigerweise geht man dabei so vor, dass man das zum Einsatz vorgesehene Benzodiazepin-Derivat der allgemeinen Formel VI kurz oder unmittelbar vor der Umsetzung mit der Aminoverbindung der Formel VII auf die weiter unten beschriebene Weise aus einem entsprechenden Benzodiazepin-Derivat der allgemeinen Formel Ia herstellt und das betreffende Isocyanat der allgemeinen Formel VI nicht in isolierter Form in die Reaktion einbringt, sondern in der Lösung, in welcher es vorgängig aus dem entsprechenden Benzodiazepin-Derivat der allgemeinen Formel Ia hergestellt worden ist.

Zu der erwähnten, das Benzodiazepin-Derivat der allgemeinen Formel VI enthaltenden Lösung kann man dann eine Aminoverbindung der Formel VII geben. Dabei kann die Aminoverbindung in Form einer Lösung oder auch ohne Lösungsmittel verwendet werden; falls es sich um eine bei Raumtemperatur gasförmige Aminoverbindung handelt (was beispielsweise für Methylamin zutrifft) kann man sie als Gas in die erwähnte Lösung des Isocyanates der Formel VI einleiten.

Andererseits ist es auch möglich, die Aminoverbindung vorzulegen, zweckmässigerweise in Form einer Lösung, und dann die erwähnte Lösung des Isocyanates der Formel VI zuzugeben.

In vielen Fällen ist es zweckmässig, die Aminoverbindung der Formel VII im Ueberschuss einzusetzen, und dies ist dann notwendig, wenn sie mehr als 1 Stickstoffatom enthält, welches zur Reaktion mit einer Isocyanatgruppe befähigt ist; dies ist beispielsweise beim Piperazin der Fall.

Als Lösungsmittel für den vorliegenden Verfahrensaspekt eignen sich verschiedene, unter den Reaktionsbedingungen inerte organische Lösungsmittel, beispielsweise halogenierte Kohlenwasserstoffe, wie z.B. Dichloräthan, Methylenchlorid, Chloroform und o-Dichlorbenzol, und Aether, wie z.B. Tetrahydrofuran, Dioxan, Dimethoxyäthan und Diäthylenglykoldimethyläther oder dergleichen.

Zweckmässigerweise arbeitet man bei Raumtemperatur oder Temperaturen unterhalb davon. Es ist noch zu beachten, dass man dann, wenn man eine Lösung des Isocyanates der allgemeinen Formel VI vorlegt, die Aminoverbindung der Formel VII innerhalb kurzer Zeit zugeben sollte, wogegen im umgekehrten Fall, d.h. wenn man die Aminoverbindung der Formel VII vorlegt und dann die Lösung des Isocyanates der allgemeinen Formel II zugibt, die Geschwindigkeit der Zugabe keine wesentliche Rolle spielt.

Gemäss Verfahrensvariante f) kann ein 3,3-Dialkylbenzodiazepin-Derivat der allgemeinen Formel I hergestellt werden, indem man eine Verbindung der allgemeinen Formel VIII mit einem den Rest $R^1$ liefernden Alkylierungsmittel behandelt. Für den vorliegenden Verfahrensaspekt kann man jedes geeignete Alkylierungsmittel verwenden. Zweckmässigerweise verwendet man dabei Halogenide, wie beispielsweise Methyljodid, Aethyljodid, Isopropylbromid, n-Propylbromid, n-Butylbromid, 2-Bromäthanol, N,N-Diäthylamino-äthylchlorid und dergleichen. Dialkylsulfate, wie z.B. Dimethylsulfat und Diäthylsulfat, oder dergleichen, und arbeitet in einem inerten organischen Lösungsmittel, beispielsweise in einem Aether, wie z.B. Tetrahydrofuran, Dioxan und Diäthyläther, oder in Aceton, N,N-Dimethylformamid oder dergleichen, in Gegenwart eines säurebindenden Mittels, wie z.B. Kalium- und Natriumcarbonat, zweckmässigerweise bei Raumtemperatur.

Gemäss Verfahrensvariante g) kann ein 3,3-Dialkylbenzodiazepin-Derivat der allgemeinen Formel I hergestellt werden, indem man eine Verbindung der allgemeinen Formel Ib halogeniert. Als Halogenierungsmittel verwendet man zweckmässigerweise Verbindungen, wie N-Chlor-succinimid, N-Brom-succinimid, N-Chlor-acetamid und dergleichen. Als Lösungsmittel für den vorliegenden Verfahrensaspekt eignen sich in erster Linie halogenierte Kohlenwasserstoffe, wie z.B. Methylenchlorid, 1,2-Dichloräthan, Chloroform und dergleichen, oder andere inerte organische Lösungsmittel, wie z.B. Acetonitril, Aether, usw. Denkbar sind aber auch wässrige Systeme, wie beispielsweise Mischungen aus konz. Salzsäure und Ameisensäure und dergleichen. Vorzugsweise arbeitet man bei Temperaturen von etwa 0°C bis Raumtemperatur, zweckmässigerweise bei Raumtemperatur.

Weitere geeignete Halogenierungsmittel sind elementares Halogen, wie Chlorgas und Brom. In diesem Fall arbeitet man vorzugsweise in saurer wässriger Lösung, wobei in manchen Fällen als Säure zweckmässigerweise der entsprechende Halogenwasserstoff verwendet wird, bei Temperaturen von etwa -10° bis +10°C, vorzugsweise bei 0°C, oder in einem sauren organischen Lösungsmittel, wie beispielsweise Ameisensäure, Essigsäure und dergleichen, zweckmässigerweise bei Raumtemperatur, oder unterhalb oder oberhalb davon.

Gemäss Verfahrensvariante h) kann ein 3,3-Dialkylbenzodiazepin-Derivat der allgemeinen Formel I hergestellt werden, indem man in einer Verbindung der allgemeinen Formel Ia die primäre Aminogruppe in die Nitrogruppe überführt. Beispielsweise kann man ein Diazoniumsalz, wie das entsprechende Diazonium-tetrafluoroborat, das aus dem Amin der Formel Ia leicht zugänglich ist und nicht isoliert werden muss, mit einem Nitrit wie Natriumnitrit, und einem Kupfer(I)salz umsetzen. Zweckmässigerweise arbeitet man in Wasser, bei Temperaturen von etwa -10°C bis Raumtemperatur.

Eine Aminoverbindung der allgemeinen Formel Ia kann aber auch durch Oxidation in die entsprechende Nitroverbindung übergeführt werden. Als Oxidationsmittel verwendet man zweckmässigerweise Wasserstoffperoxid in Essigsäure

und eine katalytische Menge einer Mineralsäure, wie Schwefelsäure. Weitere geeignete Oxidationsmittel sind beispielsweise Peressigsäure, Perbenzoesäure, Trifluorperessigsäure und dergleichen. Die notwendigen Reaktionsbedingungen können von jedem Fachmann leicht ermittelt werden.

Gemäss Verfahrensvariante i) kann ein 3,3-Dialkylbenzodiazepin-Derivat der allgemeinen Formel I hergestellt werden, indem man in einer Verbindung der allgemeinen Formel Ia die Nitrogruppe zur Aminogruppe reduziert. Zweckmässigerweise verwendet man Reduktionsmittel, wie Zinn(II)-chlorid, Zinn, Zink und dergleichen, in saurem wässrigem Milieu, beispielsweise in wässriger Salzsäure, konzentrierter Salzsäure oder dergleichen, in einem Temperaturbereich von etwa 0°C bis Raumtemperatur.

Gemäss Verfahrensvariante j) kann ein 3,3-Dialkylbenzodiazepin-Derivat der allgemeinen Formel I hergestellt werden, indem man in einer Verbindung der allgemeinen Formel Ia die primäre Aminogruppe mono- oder dialkyliert. Als Alkylierungsmittel kommen dabei Halogenide, wie Methyljodid, Isopropylbromid und n-Butylbromid, und Dialkylsulfate, wie Dimethylsulfat und Diäthylsulfat, in Kombination mit einem säurebindenden Mittel, wie Kaliumcarbonat, Natriumcarbonat, Triäthylamin, Chinuclidin und dergleichen, oder Aldehyde und Ketone, wie Formaldehyd, Acetaldehyd, Aceton, in Kombination mit einem Reduktionsmittel, wie Ameisensäure, in Frage.

Die Alkylierung erfolgt zweckmässigerweise in einem unter den Reaktionsbedingungen inerten organischen Lösungsmittel, wie Acetonitril, Diäthyläther, Tetrahydrofuran, Dimethoxyäthan und dergleichen. Führt man die Reaktion mit einem Aldehyd oder Keton in Gegenwart eines Reduktionsmittels durch, so verwendet man zweckmässigerweise Ameisensäure als Lösungs- und Reduktionsmittel. Die Temperatur für den vorliegenden Verfahrensaspekt ist nicht

kritisch, man kann demnach bei Raumtemperatur, unterhalb oder oberhalb davon, beispielsweise bei der Siedetemperatur des Reaktionsgemisches, arbeiten.

Gemäss Verfahrensvariante k) kann ein 3,3-Dialkylbenzodiazepin-Derivat der allgemeinen Formel I hergestellt werden, indem man in einer Verbindung der allgemeinen Formel Id die sekundäre Aminogruppe alkyliert. Diese Reaktion kann in Analogie zur Mono- oder Dialkylierung von Verbindungen der Formel Ia, gemäss Verfahrensvariante j), durchgeführt werden.

Gemäss Verfahrensvariante l) kann ein 3,3-Dialkylbenzodiazepin-Derivat der allgemeinen Formel I hergestellt werden, indem man eine Verbindung der allgemeinen Formel Ia mit einem Dihalogenid der Formel IX umsetzt. Diese Reaktion wird zweckmässigerweise in einem inerten organischen Lösungsmittel, wie Tetrahydrofuran, Dioxan, Acetonitril, Dimethylformamid und dergleichen, in Gegenwart eines säurebindenden Mittels, wie Kaliumcarbonat, Natriumcarbonat, Triäthylamin, Pyridin, Chinuclidin und dergleichen, durchgeführt. Vorzugsweise arbeitet man bei Raumtemperatur; man kann aber durchaus unterhalb davon, beispielsweise bei 0°C, oder oberhalb davon arbeiten.

Gemäss Verfahrensvariante m) kann ein 3,3-Dialkylbenzodiazepin-Derivat der allgemeinen Formel I hergestellt werden, indem man eine Verbindung der allgemeinen Formel Ie mit Phosgen behandelt. Besonders geeignete Lösungsmittel für den vorliegenden Verfahrensaspekt sind halogenierte Kohlenwasserstoffe, wie Methylenchlorid, Chloroform, 1,2-Dichloräthan und dergleichen, weitere geeignete Lösungsmittel sind Aether, wie t-Butyl-methyläther, Tetrahydrofuran und dergleichen, Acetonitril, Benzol, Dimethylformamid, usw. Vorzugsweise arbeitet man in einem Temperaturbereich von etwa 0°C bis Raumtemperatur.

Gemäss Verfahrensvariante n) kann ein 3,3-Dialkylbenzodiazepin-Derivat der allgemeinen Formel I hergestellt werden, indem man in einer Verbindung der allgemeinen Formel If den Aziridinring hydrolytisch öffnet.

Diese hydrolytische Ringöffnung erfolgt unter sauren Bedingungen, wobei nur Säuren in Betracht kommen, deren Anion mit dem Aziridinring nicht reagiert. Man arbeitet zweckmässigerweise in Gegenwart eines geeigneten, unter den Reaktionsbedingungen inerten organischen Lösungsmittels und bei Raumtemperatur. Beispielsweise kann man so vorgehen, dass man die Verbindung der Formel If in Dioxan oder dergleichen löst, wenig Mineralsäure (z.B. einige Tropfen 25%-ige Schwefelsäure) zugibt und das Gemisch während nicht allzu langer Zeit (z.B. 15 bis 30 Minuten) stehen lässt.

Gemäss Verfahrensvariante o) können die 3,3-Dialkylbenzodiazepin-Derivate der allgemeinen Formel I in pharmazeutisch annehmbare Säureadditionssalze übergeführt werden. Die Herstellung von solchen pharmazeutisch annehmbaren Säureadditionssalzen erfolgt nach allgemein üblichen Methoden. Es kommen sowohl Salze mit anorganischen als auch Salze mit organischen Säuren in Betracht, beispielsweise Hydrochloride, Hydrobromide, Sulfate, Citrate, Acetate, Succinate, Methansulfonate, p-Toluolsulfonate und dergleichen.

Die als Ausgangsstoffe verwendeten Verbindungen der allgemeinen Formel II können nach an sich bekannten Methoden hergestellt werden. Zweckmässigerweise geht man dabei von Verbindungen der allgemeinen Formel

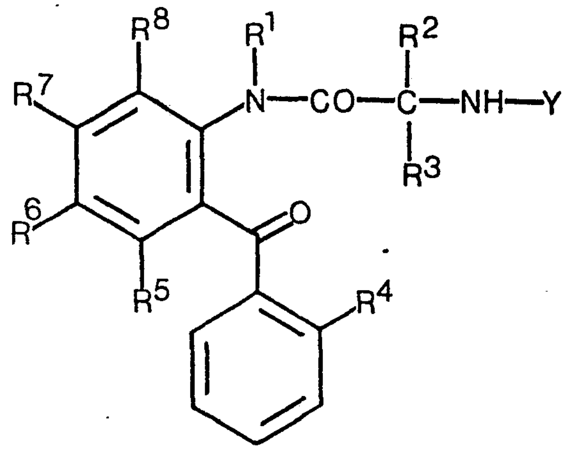

X

worin $R^1$, $R^2$, $R^3$, $R^4$, $R^5$, $R^6$, $R^7$ und $R^8$ obige Bedeutung besitzen, und Y eine Schutzgruppe bedeutet, aus, wobei als Schutzgruppen in erster Linie Acylgruppen, vorzugsweise leicht abspaltbare Alkoxycarbonyl- oder Aralkoxycarbonylgruppen, insbesondere die Benzyloxycarbonylgruppe, die unter milden sauren Bedingungen, beispielsweise mit Bromwasserstoff in Eisessig oder mit Bortrifluorid oder Bortribromid in einem interten organischen Lösungsmittel, wie Methylenchlorid oder dergleichen, abgespalten werden kann, in Frage kommen. Durch Entfernen der mit Y bezeichneten Schutzgruppe aus Verbindungen der allgemeinen Formel X erhält man Verbindungen der allgemeinen Formel II.

Verbindungen der allgemeinen Formel X können ebenfalls nach an sich bekannten Methoden hergestellt werden. Beispielsweise kann man eine Verbindung der allgemeinen Formel

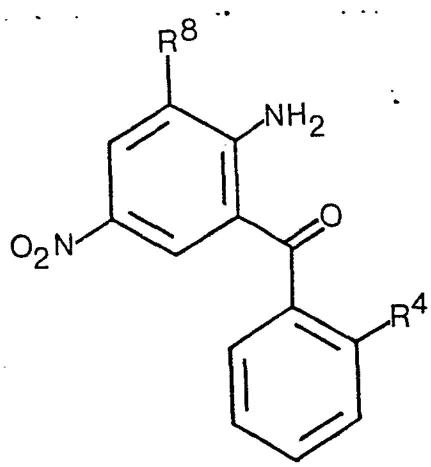

XI

worin $R^4$ und $R^8$ obige Bedeutung besitzen, in Analogie zu Verfahrensvariante l), mit einem Dihalogenid der Formel

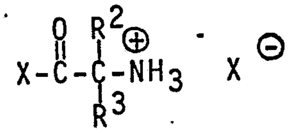

XII

worin $R^2$, $R^3$ und X obige Bedeutung besitzen, umsetzen und erhält dabei eine Verbindung der Formel

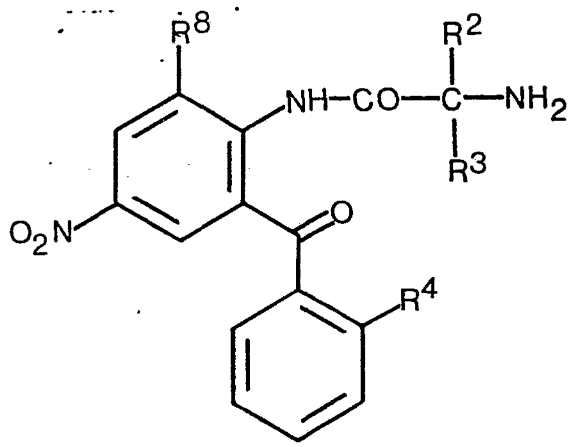

XIII

worin $R^2$, $R^3$, $R^4$ und $R^8$ obige Bedeutung besitzen. Nach Schützen der primären Aminogruppe einer Verbindung der Formel XIII mit einem geeigneten den Rest Y liefernden Reagens, wie Chlorameisensäure-benzylester oder dergleichen, und Alkylieren des erhaltenen Stoffes mit einem den Rest $R^1$ liefernden Alkylierungsmittel, in Analogie zu Verfahrensvariante f), erhält man eine Verbindung der Formel

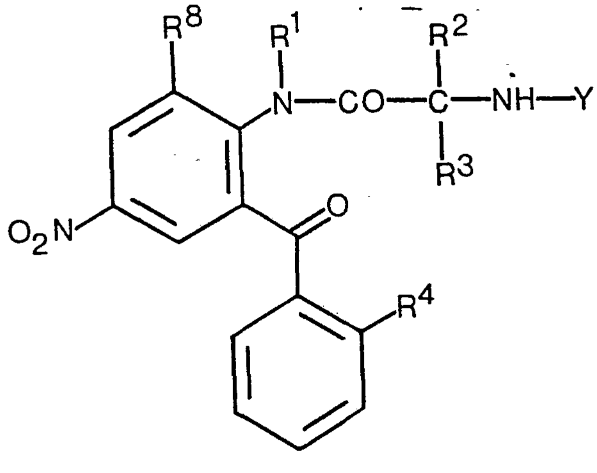

XIa

worin $R^1$, $R^2$, $R^3$, $R^4$, $R^8$ und Y obige Bedeutung besitzen.

Durch Reduktion einer solchen Verbindung in Analogie zu Verfahrensvariante i), und erwünschtenfalls Halogenierung der erhaltenen Aminoverbindung in Analogie zu Verfahrensvariante g) erhält man eine Verbindung der Formel

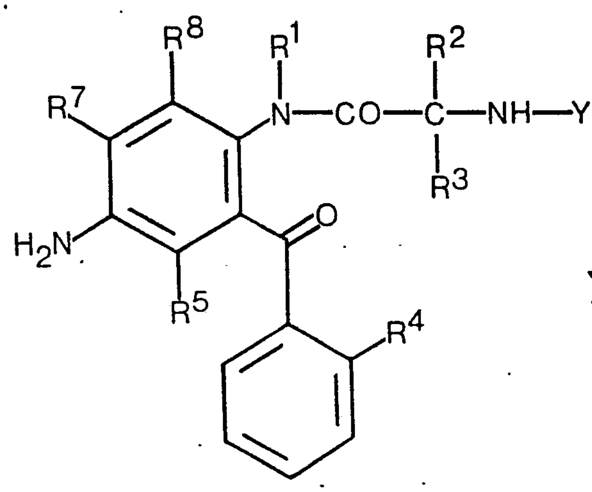

Xb

worin $R^1$, $R^2$, $R^3$, $R^4$, $R^5$, $R^7$, $R^8$ und Y obige Bedeutung besitzen.

In Analogie zu den weiter oben beschriebenen Verfahrensvarianten c), d), e), h), j), k), l), m) und n) und zur weiter unten beschriebenen Herstellung der dazu benötigten Ausgangsstoffe können Verbindungen der Formel Xb in Verbindungen der Formel X, worin $R^6$ Nitro, niederes Alkylamino, niederes Dialkylamino, oder einen Rest der Formel $R^9R^{10}N-CO-NH-$, $H_2N-C(CH_3)_2-CO-NH-$ oder

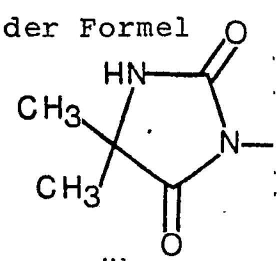

bedeuten, und $R^9$ und $R^{10}$ obige Bedeutung besitzen, übergeführt werden.

Verbindungen der Formel II können aber auch hergestellt werden, indem aus einer Verbindung der Formel

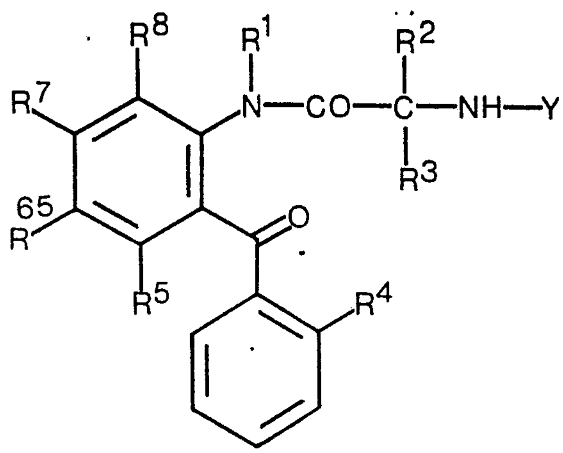

XIV

worin $R^{65}$ geschütztes niederes Alkylamino oder einen Rest der Formel $Z-NH-C(CH_3)_2-CO-NH-$ oder $R^{91}R^{101}N-CO-NH-$ bedeuten, und $R^1$, $R^2$, $R^3$, $R^4$, $R^5$, $R^7$, $R^8$, $R^{91}$, $R^{101}$, Y und Z obige Bedeutung besitzen,

die mit Y bezeichnete Schutzgruppe abspaltet, und vorgängig oder im gleichen Arbeitsgang die im Rest $R^{65}$ vorhandene(n) Schutzgruppen abspaltet, in Analogie zu Verfahrensvariante b).

Verbindungen der allgemeinen Formel XIV können in an sich bekannter Weise aus Verbindungen der Formel Xb erhalten werden. Beispielsweise indem man eine Verbindung der Formel Xb mit einem Carbamoylhalogenid der Formel

$$R^{93}R^{103}N-CO-X \qquad XV$$

worin entweder $R^{93}$ eine Schutzgruppe und $R^{103}$ niederes Alkyl oder einen Rest der Formel -A-O-Z bedeuten, oder $R^{93}$ und $R^{103}$ zusammen mit dem Stickstoffatom einen 5- bis 7-gliedrigen Heterocyclus bedeuten, der einen Rest der Formel $\rangle N-R^{01}$ als Ringglied enthält, und A, X, Z und $R^{01}$ obige Bedeutung besitzen, in Analogie zu Verfahrensvariante c),

oder mit einem Isocyanat der Formel

$$R^{104}-NCO \qquad XVI$$

worin $R^{104}$ einen Rest der Formel -A-O-Z bedeutet, und A und Z obige Bedeutung besitzen, in Analogie zu Verfahrensvariante d),

oder mit einem Carbonsäurehalogenid der Formel

$$Z\text{-}NH\text{-}C(CH_3)_2\text{-}CO\text{-}X \qquad XVII$$

worin X und Z obige Bedeutung besitzen,

in Analogie zu Verfahrensvariante l), umsetzt, oder ein Isocyanat der Formel

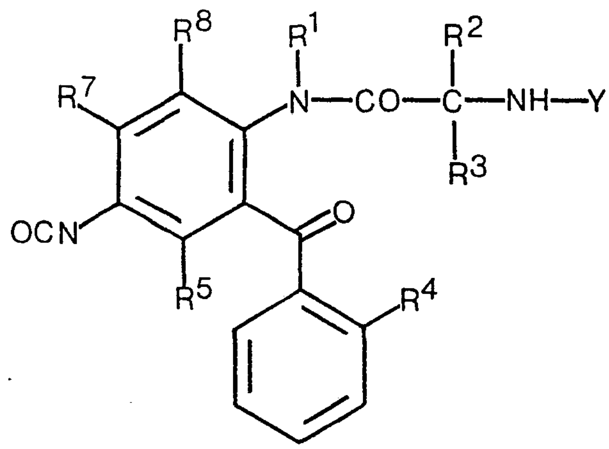

XVIII

worin $R^1$, $R^2$, $R^3$, $R^4$, $R^5$, $R^7$, $R^8$ und Y obige Bedeutung besitzen,

das aus einem Amin der Formel Xb in Analogie zur weiter unten beschriebenen Herstellung von Verbindungen der allgemeinen Formel VI aus Verbindungen der allgemeinen Formel Ia hergestellt werden kann, mit einem Amin der Formel

$$R^{91}R^{101}NH \qquad XIX$$

worin $R^{91}$ und $R^{101}$ obige Bedeutung besitzen,

in Analogie zu Verfahrensvariante e), umsetzt, oder die Aminogruppe in einer Verbindung der Formel Xb mit einem den Rest Z liefernden Mittel schützt und in Analogie zu Verfahrensvariante f) bzw. j), je nach Beschaffenheit der Schutzgruppe, alkyliert.

Eine weitere Möglichkeit zur Herstellung von Verbindungen der allgemeinen Formel II besteht darin, dass man ein Nitrobenzophenon-Derivat der Formel XI in eine Verbindung der Formel

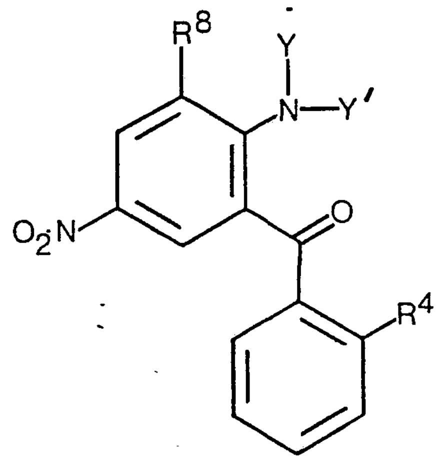

XX

worin $R^4$, $R^8$ und Y obige Bedeutung besitzen, und Y' Wasserstoff oder eine Schutzgruppe bedeutet, wobei Y zusammen mit Y' eine Schutzgruppe bedeuten kann,

überführt, hierauf die Nitrogruppe in Analogie zu Verfahrensvariante i) reduziert, und erwünschtenfalls einen so erhaltenen Stoff in Analogie zu Verfahrensvariante g) halogeniert. Die auf die soeben beschriebene Weise erhaltenen Verbindungen entsprechen der allgemeinen Formel

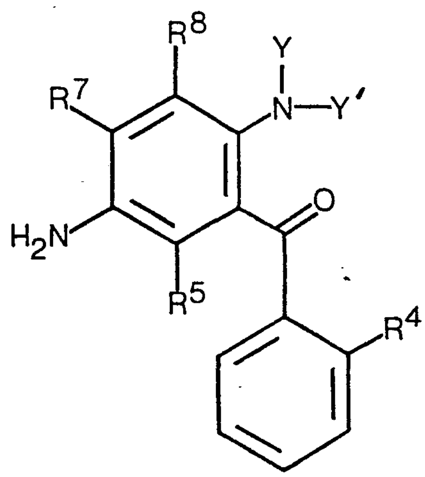

XXI

worin $R^4$, $R^5$, $R^7$, $R^8$, Y und Y' obige Bedeutung besitzen.

Eine Verbindung der obigen Formel XXI kann sodann nach an sich bekannten und teilweise weiter oben beschriebenen Methoden in eine Verbindung der Formel

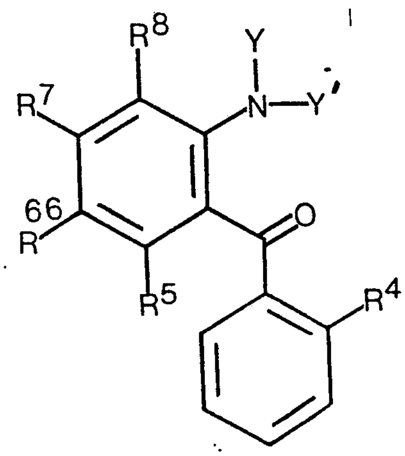

XXII

worin $R^{66}$ Nitro, niederes Dialkylamino, geschütztes niederes Alkylamino oder einen Rest der Formel ZZ'N- bedeutet, und $R^4$, $R^5$, $R^7$, $R^8$, Y und Y' obige Bedeutung besitzen, wobei Y' nur Wasserstoff bedeuten kann, wenn $R^{66}$ einen Rest der Formel ZZ'N- bedeutet,

übergeführt werden. Durch Entfernen der mit Y' und/oder Y bezeichneten Schutzgruppe(n) aus einer Verbindung der obigen allgemeinen Formel XXII, ohne andere im Molekül vorhandene Strukturelemente in Mitleidenschaft zu ziehen, erhält man ein Benzophenon-Derivat der allgemeinen Formel

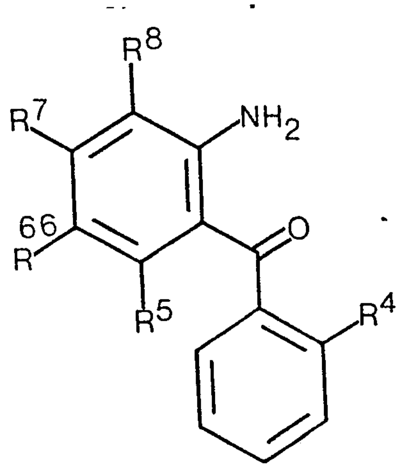

XXIII

worin $R^4$, $R^5$, $R^{66}$, $R^7$ und $R^8$ obige Bedeutung besitzen. Verbindungen der Formel XXIII können sodann, beispielsweise in Analogie zur Herstellung von Verbindungen der Formel Xa aus solchen der Formel XI, in Verbindungen der allgemeinen Formel

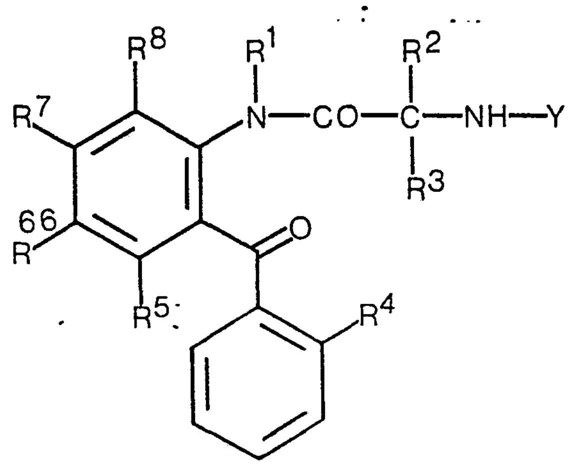

XXIV

worin $R^1$, $R^2$, $R^3$, $R^4$, $R^5$, $R^{66}$, $R^7$, $R^8$ und Y obige Bedeutung besitzen,

übergeführt werden.

Durch Abspalten der mit Y bezeichneten Schutzgruppe - und vorgängig oder im gleichen Arbeitsgang - Abspalten der im Rest $R^{66}$ allfällig vorhandenen Schutzgruppe(n) erhält man nun aus Verbindungen der allgemeinen Formel XXIV solche der allgemeinen Formel II.

Die als Ausgangsstoffe verwendeten Verbindungen der allgemeinen Formel III können beispielsweise hergestellt werden, indem man aus Verbindungen der allgemeinen Formel XIV und aus solchen der allgemeinen Formel XXIV, worin $R^{66}$ geschütztes niederes Alkylamino oder einen Rest der Formel ZZ'N- bedeutet, die mit Y bezeichnete Schutzgruppe selektiv abspaltet und den erhaltenen Stoff in Analogie zu Verfahrensvariante a) cyclisiert.

Verbindungen der allgemeinen Formel III können aber auch hergestellt werden, indem man eine Verbindung der

allgemeinen Formel XXIII, worin $R^{66}$ geschütztes niederes Alkylamino oder einen Rest der Formel ZZ'N- bedeutet, mit einem Dihalogenid der allgemeinen Formel XII umsetzt, in Analogie zu Verfahrensvariante 1), das erhaltene Produkt in Analogie zu Verfahrensvariante a) cyclisiert und eine so erhaltene Verbindung der allgemeinen Formel

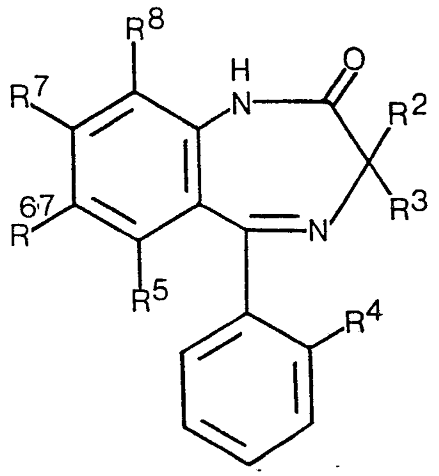

XXV

worin $R^{67}$ geschütztes niederes Alkylamino oder einen Rest der Formel ZZ'N- bedeutet, und $R^2$, $R^3$, $R^4$, $R^5$, $R^7$ und $R^8$ obige Bedeutung besitzen,

mit einem geeigneten den Rest $R^1$ liefernden Alkylierungsmittel behandelt (in Analogie zu Verfahrensvariante f)).

Eine weitere Möglichkeit zur Herstellung von Verbindungen der allgemeinen Formel III besteht darin, dass man eine Verbindung der allgemeinen Formel Ia mit einem Carbamoylhalogenid der Formel XV oder einem Isocyanat der Formel XVI in Analogie zu den Verfahrensvariante c) bzw. d), oder in Analogie zu Verfahrensvariante 1) mit einem Carbonsäurehalogenid der Formel XVIII umsetzt, oder ein Isocyanat der allgemeinen Formel VI, dessen Herstellung weiter unten beschrieben wird, in Analogie zu Verfahrensvariante e) mit einem Amin der Formel XIX umsetzt, oder in einer Verbindung der allgemeinen Formel Ia die primäre Aminogruppe geeignet schützt und in Analogie zu Verfahrensvariante f) bzw. j), je nach Beschaffenheit der verwendeten Schutzgruppe, alkyliert.

Die als Ausgangsstoffe verwendeten Verbindungen der allgemeinen Formel VI können - wie bereits weiter oben erwähnt - aus entsprechenden Verbindungen der allgemeinen Formel Ia hergestellt werden, und zwar durch Umsetzen mit Phosgen. Dabei geht man zweckmässigerweise so vor, dass man eine Lösung von Phosgen in einem unter den Reaktionsbedingungen inerten organischen Lösungsmittel vorlegt und dann unter Kühlen eine Lösung einer Verbindung der allgemeinen Formel Ia zugibt, hierauf einige Zeit unter Rückfluss zum Sieden erhitzt, dann wieder abkühlt und schliesslich die erhaltene Lösung mit einer tertiären organischen Aminoverbindung, wie Triäthylamin, basisch oder mindestens neutral stellt. Die erhaltene Lösung, enthaltend eine Verbindung der allgemeinen Formel VI, kann unter Feuchtigkeitsausschluss und in der Kälte während mehreren Stunden aufbewahrt werden; sie wird - wie weiter oben ausgeführt - ohne Isolierung der darin enthaltenen Verbindung der allgemeinen Formel VI direkt weiterverarbeitet.

Die als Ausgangsstoffe verwendeten Verbindungen der allgemeinen Formel VIII sind bekannt oder können nach an sich bekannten Methoden hergestellt werden, beispielsweise indem man eine Verbindung der allgemeinen Formel XIII in Analogie zu Verfahrensvariante a) cyclisiert und erwünschtenfalls die Nitrogruppe im erhaltenen Stoff in Analogie zu Verfahrensvariante i) zur Aminogruppe reduziert und diese in Analogie zu Verfahrensvariante j) dialkyliert.

Verbindungen der allgemeinen Formel VIII können aber auch hergestellt werden, indem man eine Verbindung der Formel XXIII, worin $R^{66}$ niederes Dialkylamino bedeutet, wie oben beschrieben, mit einer Verbindung der Formel XII umsetzt, und das erhaltene Produkt cyclisiert.

Eine weitere Möglichkeit zur Herstellung von Verbindungen der allgemeinen Formel VIII besteht darin, dass man aus einer Verbindung der Formel XXV die im Rest $R^{67}$ vorhandene(n) Schutzgruppe(n) entfernt, und die erhaltene

Aminoverbindung in Analogie zu Verfahrensvariante j) bzw. k) alkyliert.

Die als Ausgangsmaterialien verwendeten Verbindungen der allgemeinen Formeln II, III und VI sind neu und ebenfalls Gegenstand der vorliegenden Erfindung.

Ueberraschenderweise hat es sich gezeigt, dass die 3,3-Dialkylbenzodiazepin-Derivate der eingangs definierten allgemeinen Formel I keine oder nur sehr geringe Wirkungen auf das zentrale Nervensystem entfalten, wogegen sie ausgeprägte aldosteronantagonistische Eigenschaften aufweisen. Diese aldosteronantagonistischen Eigenschaften lassen sich, wie nachstehend erläutert, an adrenalektomierten Ratten nachweisen.

Verabreicht man andrenalektomierten Ratten Aldosteron, so beobachtet man - im Vergleich zu unbehandelten Tieren - eine ausgeprägte Verminderung der Natrium-Ausscheidung (Natrium-Retention), eine erhöhte Kalium-Ausscheidung (Kalium-Exkretion) sowie eine Verminderung des ausgeschiedenen Harnvolumens. Gibt man den Tieren vor der Behandlung mit Aldosteron Verbindungen der Formel I, so beobachtet man - im Vergleich zu nur mit Aldosteron behandelten Tieren (Kontrolltiere) - eine ausgeprägte Erhöhung der Natrium-Ausscheidung (das heisst: die durch Aldosteron bewirkte Natrium-Retention wird antagonisiert), wogegen die Kalium-Exkretion und das Harnvolumen in geringerem Ausmass beeinfluss werden.

Der Standardversuch wird wie folgt durchgeführt:

Weibliche Holtzmann-Ratten (150-180 g) werden 70 bis 74 Stunden vor Versuchsbeginn bilateral adrenalektomiert. Nach der Operation erhalten die Tiere ein gebräuchliches Ratten-Trockenfutter und 0,9%ige Kochsalzlösung zum Trinken. 16-17 Stunden vor Versuchsbeginn wird den Tieren das Futter entzogen, wogegen sie nach wie vor ad libitum 0,9%ige

Kochsalzlösung trinken können. Bei Versuchsbeginn wird den Tieren die auf Aldosteronantagonismus zu prüfende Substanz mittels einer Magensonde verabreicht. 30 Minuten später erhalten die Tiere eine subkutane Injektion von 4 mmg/kg Aldosteron. Nach weiteren 90 Minuten werden die Harnblasen der Tiere durch sorgfältiges suprapubisches Drücken entleert, worauf die Tiere ohne Nahrung und ohne Getränk einzeln in Metabolismuskäfige verbracht werden. Der Urin der Tiere wird dann während 3 Stunden gesammelt, worauf ihre Harnblasen nochmals entleert werden. Der spontan ausgeschiedene Harn und der am Schluss des Versuches durch Ausdrücken der Harnblasen erhaltene Restharn werden in graduierten Zentrifugengläsern gesammelt. Natrium- und Kalium-Konzentrationen des Harns werden mit einem Flammenphotometer bestimmt.

In der nachfolgenden Tabelle werden die Resultate dargestellt, welche mit repräsentativen Vertretern der durch die allgemeine Formel I definierten Verbindungsklasse in dem vorstehend geschilderten Versuch erhalten worden sind. Angegeben werden in dieser Tabelle für jede der darin figurierenden Verbindung die verabreichte Dosis ( in mg/kg p.o.) sowie die prozentuale Veränderung des Harnvolumens, der Natrium-Ausscheidung und der Kalium-Ausscheidung im Vergleich zu den Kontrolltieren (das heisst im Vergleich zu den nur mit Aldosteron behandelten Tieren). Ausserdem enthält die Tabelle Angaben über die akute Toxizität der untersuchten Verbindungen (DL 50 in mg/kg bei einmaliger oraler Verabreichung an Mäuse).

Toxizität und Wirkung an der adrenalektomierten Ratte

| $R^1$ | $R^2$ | $R^3$ | $R^4$ | $R^5$ | $R^7$ | $R^8$ | $R^6$ | Dosis in mg/kg p.o. | Volumen | $[Na^{\oplus}]$ | $[K^{\oplus}]$ | DL 50 in mg/kg p.o. |
|---|---|---|---|---|---|---|---|---|---|---|---|---|
| | | | | | | | | | in % bez. auf Kontrolltiere | | | |
| $-CH_3$ | $-CH_3$ | $-CH_3$ | -F | -H | -H | -Cl | $-NH_2$ | 0,01 | 120 | 201 | 80 | >5000 |
| $-CH_3$ | $-CH_3$ | $-CH_3$ | -F | -Br | -Br | -H | $-NH_2$ | 1 | 161 | 281 | 76 | >5000 |
| $-CH_3$ | $-CH_3$ | $-CH_3$ | -Cl | -Br | -Br | -H | $-NH_2$ | 0,1 | 138 | 361 | 168 | >5000 |
| $-CH_3$ | $-CH_3$ | $-CH_3$ | -Cl | -Cl | -Cl | -H | $-NH_2$ | 0,1 | 98 | 215 | 85 | >5000 |
| $-CH_3$ | $-CH_3$ | $-CH_3$ | -F | -Cl | -H | -H | $-NH_2$ | 0,01 | 111 | 217 | 83 | >5000 |
| $-CH_3$ | $-CH_3$ | $-CH_3$ | -Cl | -H | -H | -H | $-NHCONH(CH_2)_2OH$ | 0,1 | 131 | 309 | 76 | >5000 |
| $-CH_3$ | $-CH_3$ | $-CH_3$ | -F | -H | -H | -H | $-NHCOC(CH_3)_2NH_2$ | 0,1 | 101 | 246 | 102 | 1250-2500 |
| $-CH_3$ | $-CH_3$ | $-CH_3$ | -F | -Cl | -Cl | -Cl | $-NH_2$ | 1 | 124 | 208 | 108 | >5000 |
| $-CH_3$ | $-CH_3$ | $-CH_3$ | -F | -H | -Cl | -Cl | $-NH_2$ | 1 | 104 | 209 | 86 | - |
| $-CH_3$ | $-CH_3$ | $-CH_3$ | -Cl | -H | -H | -Cl | $-NH_2$ | 1 | 123 | 209 | 117 | >5000 |
| $-CH_3$ | $-CH_3$ | $-CH_3$ | -Cl | -Br | -H | -H | $-NHCONH(CH_2)_2OH$ | 1 | 210 | 233 | 99 | >5000 |

Die Benzodiazepin-Derivate der allgemeinen Formel I und ihre pharmazeutisch annehmbaren Säureadditionssalze können als Heilmittel, z.B. in Form pharmazeutischer Präparate, Verwendung finden. Die pharmazeutischen Präparate können oral, z.B. in Form von Tabletten, Lacktabletten, Dragées, Hart- und Weichgelatinekapseln, Lösungen, Emulsionen oder Suspensionen, verabreicht werden. Die Verabreichung kann aber auch rektal, z.B. in Form von Suppositorien, oder parenteral, z.B. in Form von Injektionslösungen, erfolgen.

Zur Herstellung von Tabletten, Lacktabletten, Dragées und Hartgelatinekapseln können die Benzodiazepin-Derivate der allgemeinen Formel I und ihre pharmazeutisch annehmbaren Säureadditionssalze mit pharmazeutisch inerten, anorganischen und/oder organischen Trägern verabreicht werden. Als Träger kann man für Tabletten, Dragées und Hartgelatinekapseln z.B. Lactose, Maisstärke oder Derivate davon, Talk, Stearinsäure oder deren Salze etc. verwenden.

Für Weichgelatinekapseln eignen sich als Träger pflanzliche Oele, Wachse, Fette, halbfeste und flüssige Polyole etc.; je nach Beschaffenheit des Wirkstoffes sind jedoch bei Weichgelatinekapseln überhaupt keine Träger erforderlich.

Zur Herstellung von Lösungen und Sirupen eignen sich als Träger z.B. Wasser, Polyole, Saccharose, Invertzucker, Glukose und dergleichen.

Für Injektionslösungen eignen sich als Träger Wasser, Alkohole, Polyole, Glyzerin, pflanzliche Oele und dergleichen.

Für Suppositorien eignen sich als Träger z.B. natürliche oder gehärtete Oele, Wachse, Fette, halbflüssige oder flüssige Polyole und dergleichen.

Die pharmazeutischen Präparate können daneben auch noch andere therapeutisch wertvolle Stoffe und/oder Hilfsstoffe, wie Konservierungsmittel, Lösevermittler, Stabilisatoren, Netzmittel, Emulgatoren, Süssmittel, Färbemittel, Aromatisierungsmittel, Salze zur Veränderung des osmotischen Druckes, Puffer, Ueberzugsmittel oder Antioxidantien enthalten.

Wie eingangs erwähnt sind Arzneimittel, enthaltend eine oder mehrere Verbindungen der allgemeinen Formel I oder pharmazeutisch annehmbare Säureadditionssalze davon, ebenfalls Gegenstand der vorliegenden Erfindung, weiterhin auch ein Verfahren zur Herstellung solcher Arzneimittel, welches dadurch gekennzeichnet ist, dass man eine oder mehrere Verbindungen der allgemeinen Formel I oder pharmazeutisch annehmbare Säureadditionssalze davon in eine galenische Darreichungsform bringt. Ein weiterer Gegenstand der vorliegenden Erfindung ist - wie eingangs erwähnt - die Verwendung von 3,3-Dialkylbenzodiazepin-Derivaten der allgemeinen Formel I und von pharmazeutisch annehmbaren Säureadditionssalzen davon bei der Bekämpfung bzw. Verhütung von Krankheiten, insbesondere bei der Bekämpfung bzw. Verhütung von Herzversagen, von hepatitischer Aszites, von primärem Aldosteronismus und von essentieller Hypertonie. Die Dosierung kann innerhalb weiter Grenzen variieren und ist natürlich in jedem einzelnen Fall den individuellen Gegebenheiten anzupassen. Im Allgemeinen dürfte bei oraler Verabreichung eine Tagesdosis von etwa 20 mg bis 1500 mg angemessen sein.

In den nachfolgenden Beispielen, welche die vorliegende Erfindung näher erläutern, ihren Umfang jedoch in keiner Weise beschränken sollen, sind sämtliche Temperaturen in Celsiusgraden angegeben.

Beispiel 1

a) Eine Lösung von 206 g (2 Mol) α-Amino-isobuttersäure in 1,2 l absolutem Tetrahydrofuran wird mit 240 ml Thionylchlorid versetzt und 24 Stunden bei Raumtemperatur gerührt. Nach Zugabe von 520 g 2-Amino-5-nitro-o'-fluorbenzophenon in 2 l abs. Tetrahydrofuran rührt man das Reaktionsgemisch 3 Tage bei Raumtemperatur, engt auf etwa 2/3 des Gesamtvolumens ein. Man filtriert das ausgefallene Hydrochlorid ab, versetzt mit Eiswasser und Methylenchlorid und stellt die wässrige Phase mit Ammoniak alkalisch. Anschliessend wird die wässerige Phase dreimal mit Methylenchlorid, das 5% Aethanol enthält, ausgeschüttelt. Nach Trocknen und Eindampfen der vereinigten organischen Phasen erhält man rohes 2-Amino-2'-(o-fluorbenzoyl)-2-methyl-4'-nitro-propionanilid, das direkt weiterverarbeitet wird. Eine Probe des Materials wird aus Methylenchloird/Aether umkristallisiert und schmilzt bei 154°.

b) Eine Lösung von 690 g (2 Mol) des obigen Zwischenproduktes in 3,6 l Toluol und 450 ml Eisessig wird am Wasserabscheider zum Sieden erhitzt. Nach beendeter Wasserabspaltung engt man das Reaktionsgemisch ein und lässt abkühlen. Das auskristallisierte Produkt wird abfiltriert, nacheinander mit Toluol und Aether gewaschen und bei Raumtemperatur im Wasserstrahlvakuum getrocknet. Man erhält 5-(o-Fluorphenyl)-1,3-dihydro-3,3-dimethyl-7-nitro-2H-1,4-benzodiazepin-2-on vom Schmelzpunkt 243-244°.

c) 18,2 g (0,056 Mol) 5-(o-Fluorphenyl)-1,3-dihydro-3,3-dimethyl-7-nitro-2H-1,4-benzodiazepin-2-on werden in 150 ml trockenem Aceton gelöst, mit 18 g gemahlenem Kaliumcarbonat und 9 ml Methyljodid versetzt und 2 1/2 Stunden bei Raumtemperatur gerührt. Anschliessend wird das Reaktionsgemisch eingedampft und der Rückstand zwischen Methylenchlorid und Wasser verteilt. Nach Abtrennen der organischen Phase wird noch mehrmals mit Methylenchlorid extrahiert; die vereinigten organischen Extrakte werden getrocknet

und eingedampft. Der Rückstand wird aus Benzol/Hexan/Petroläther umkristallisiert und ergibt 5-(o-Fluorphenyl)-1,3-dihydro-1,3,3-trimethyl-7-nitro-2H-1,4-benzodiazepin-2-on vom Schmelzpunkt 128-129°.

Beispiel 2

1,5 g (0,0044 Mol) 5-(o-Fluorphenyl)-1,3-dihydro-1,3,3-trimethyl-7-nitro-2H-1,4-benzodiazepin-2-on werden in 15 ml konz. Salzsäure gelöst, mit 3,3 g Zinn(II)chlorid versetzt und 15 Minuten bei Raumtemperatur gerührt. Das Reaktionsgemisch wird auf eine Mischung aus Eis und Sodalösung gegossen und mit Methylenchlorid extrahiert. Nach Trocknen und Eindampfen der organischen Phase wird der Rückstand aus Aether umkristallisiert. Man erhält 7-Amino-5-(o-fluorphenyl)-1,3-dihydro-1,3,3-trimethyl-2H-1,4-benzodiazepin-2-on vom Schmelzpunkt 190-191°.

Beispiel 3

Durch eine Lösung von 6 g (0,019 Mol) 7-Amino-5-(o-fluorphenyl)-1,3-dihydro-1,3,3-trimethyl-2H-1,4-benzodiazepin-2-on in 70 ml konz. Salzsäure leitet man zwischen -5 bis 0° langsam so lange Chlorgas ein, bis das Ausgangsmaterial fast ganz aufgebraucht ist. Das Reaktionsgemisch wird auf eine Mischung aus Eis und Sodalösung gegossen, worauf mit Methylenchlorid extrahiert und der Extrakt getrocknet und eingedampft wird. Der Rückstand wird an 300 g Kieselgel mit Methylenchlorid/Essigester (10:1) als Elutionsmittel chromatographiert. Aus Aether/Petroläther erhält man 7-Amino-6-chlor-5-(o-fluorphenyl)-1,3-dihydro-1,3,3-trimethyl-2H-1,4-benzodiazepin-2-on vom Schmelzpunkt 192-193°.

Beispiel 4

a) Eine Lösung von 3,5 g (0,01 Mol) 7-Amino-6-chlor-5-(o-fluorphenyl)-1,3-dihydro-1,3,3-trimethyl-2H-1,4-benzodiazepin-2-on in 60 ml 1,2-Dichloräthan wird unter Rühren so zu

einer Lösung von 1,5 g (0,015 Mol) Phosgen in eisgekühltem 1,2-Dichloräthan zugetropft, dass die Reaktionstemperatur 10° nicht übersteigt. Anschliessend wird das Reaktionsgemisch noch 10 Minuten unter Rühren am Rückfluss erhitzt, worauf der Ansatz auf ca. 10° gekühlt und mit Triäthylamin basisch gestellt wird. Dabei erhält man eine Lösung von [6-Chlor-5-(o-fluorphenyl)-2,3-dihydro-1,3,3-trimethyl-2-oxo-1H-1,4-benzodiazepin-7-yl]isocyanat, die unter Feuchtigkeitsausschluss mehrere Stunden in der Kälte aufbewahrt werden kann. Sie wird ohne Isolierung des darin enthaltenen Isocyanats weiterverarbeitet.

b) Die oben erhaltene Lösung des Isocyanates gibt man zu 2 ml Aethanolamin in 1,2-Dichloräthan und dampft das Reaktionsgemisch ein. Der Rückstand wird an 150 g Kieselgel mit Methylenchlorid, Methylenchlorid/Essigester (1:1) und Essigester als Elutionsmittel gereinigt. Aus Aceton/Aether erhält man 1-[6-Chlor-5-(o-fluorphenyl)-2,3-dihydro-1,3,3-trimethyl-2-oxo-1H-1,4-benzodiazepin-7-yl]-3-(2-hydroxyäthyl)harnstoff vom Schmelzpunkt 146-147° (Zersetzung).

Beispiel 5

6,6 g (0,021 Mol) 7-Amino-5-(o-fluorphenyl)-1,3-dihydro-1,3,3-trimethyl-2H-1,4-benzodiazepin-2-on, gelöst in 50 ml konz. wässeriger Bromwasserstoffsäure, werden zwischen 0 bis 5° langsam mit 1,2 ml Brom versetzt und 1 Stunde bei 0° gerührt. Anschliessend giesst man das Reaktionsgemisch auf eine Mischung aus Eis und Sodalösung, extrahiert mit Methylenchlorid und dampft den organischen Auszug ein. Der Rückstand wird an 300 g Kieselgel mit Methylenchlorid/Essigester (20:1) gereinigt. Aus Essigester/Aether erhält man 7-Amino-6-brom-5-(o-fluorphenyl)-1,3-dihydro-1,3,3-trimethyl-2H-1,4-benzodiazepin-2-on vom Schmelzpunkt 217-220°.

## Beispiel 6

Aus 2,1 g (0,0054 Mol) 7-Amino-6-brom-5-(o-fluorphenyl)-1,3-dihydro-1,3,3-trimethyl-2H-1,4-benzodiazepin-2-on erhält man, in Analogie zu den Angaben in Beispiel 4 1-[6-Brom-5-(o-fluorphenyl)-2,3-dihydro-1,3,3-trimethyl-2-oxo-1H-1,4-benzodiazepin-7-yl]-3-(2-hydroxyäthyl)harnstoff vom Schmelzpunkt 202° (Aethanol/Essigester/n-Hexan).

## Beispiel 7

a) Aus 70 g (0,25 Mol) 2-Amino-5-nitro-2'-chlor-benzophenon erhält man, in Analogie zu den Angaben in Absatz a) von Beispiel 1, 2-Amino-2'-(o-chlorbenzoyl)-2-methyl-4'-nitro-propionanilid vom Schmelzpunkt 138-139° (Aether/Petroläther).

b) Aus 76 g (0,21 Mol) 2-Amino-2'-(o-chlorbenzoyl)-2-methyl-4'-nitro-propionanilid erhält man, in Analogie zu den Angaben in Absatz b) von Beispiel 1, 5-(o-Chlorphenyl)-1,3-dihydro-3,3-dimethyl-7-nitro-2H-1,4-benzodiazepin-2-on vom Schmelzpunkt 242° (Methylenchlorid/n-Hexan).

c) Aus 59 g (0,17 Mol) 5-(o-Chlorphenyl)-1,3-dihydro-3,3-dimethyl-7-nitro-2H-1,4-benzodiazepin-2-on erhält man, in Analogie zu den Angaben in Absatz c) von Beispiel 1, 5-(o-Chlorphenyl)-1,3-dihydro-1,3,3-trimethyl-7-nitro-2H-1,4-benzodiazepin-2-on vom Schmelzpunkt 125-127° (Methylenchlorid/Essigester).

## Beispiel 8

Aus 44,5 g (0,124 Mol) 5-(o-Chlorphenyl)-1,3-dihydro-1,3,3-trimethyl-7-nitro-2H-1,4-benzodiazepin-2-on erhält man, in Analogie zu den Angaben in Beispiel 2, 7-Amino-5-(o-chlorphenyl)-1,3-dihydro-1,3,3-trimethyl-2H-1,4-benzodiazepin-2-on vom Schmelzpunkt 177-178° (Aether/n-Hexan).

## Beispiel 9

Aus 8 g (0,024 Mol) 7-Amino-5-(o-chlorphenyl)-1,3-dihydro-1,3,3-trimethyl-2H-1,4-benzodiazepin-2-on erhält man in Analogie zu den Angaben in Beispiel 4, über das [5-(o-Chlorphenyl)-2,3-dihydro-1,3,3-trimethyl-2-oxo-1H-1,4-benzodiazepin-7-yl]isocyanat, 1-[5-(o-Chlorphenyl)-2,3-dihydro-1,3,3-trimethyl-2-oxo-1H-1,4-benzodiazepin-7-yl]-3-(2-hydroxyäthyl)harnstoff vom Schmelzpunkt 85-110° (Essigester/n-Hexan).

## Beispiel 10

17 g (0,052 Mol) 7-Amino-5-(o-chlorphenyl)-1,3-dihydro-1,3,3-trimethyl-2H-1,4-benzodiazepin-2-on in 100 ml Eisessig werden mit einer Lösung von 4 g Chlorgas in 50 ml Eisessig langsam versetzt. Das Reaktionsgemisch wird bei Raumtemperatur gerührt, nach 60 Minuten eingedampft und der Rückstand wird mit wässrigem Ammoniak und Methylenchlorid versetzt. Nach Ausschütteln der wässrigen Phase mit Methylenchlorid, wird der organische Auszug getrocknet und eingedampft. Das Rohprodukt wird durch Chromatographie an 600 g Kieselgel mit Methylenchlorid und Methylenchlorid/Essigester (5:1) als Elutionsmittel gereinigt. Aus Essigester/Aether/n-Hexan erhält man 7-Amino-6-chlor-5-(o-chlorphenyl)-1,3-dihydro-1,3,3-trimethyl-2H-1,4-benzodiazepin-2-on vom Schmelzpunkt 195-196°.

## Beispiel 11

16 g (0,049 Mol) 7-Amino-5-(o-chlorphenyl)-1,3-dihydro-1,3,3-trimethyl-2H-1,4-benzodiazepin-2-on in 160 ml Eisessig versetzt man unter Rühren langsam mit 8,5 g Brom in 10 ml Eisessig. Nach 60 Minuten bei Raumtemperatur wird das Reaktionsgemisch eingeengt und der Rückstand zwischen wässrigem Ammoniak und Methylenchlorid verteilt. Die wässrige Phase wird noch mehrmals mit Methylenchlorid ausgeschüttelt. Die vereinigten organischen Auszüge werden getrocknet und eingedampft. Das Rohprodukt wird an 500 g Kieselgel mit Methylenchlorid/Essigester (5:1) als Elutionsmittel gereinigt. Aus Essigester/Aether erhält man 7-Amino-6-brom-5-(o-chlorphenyl)-1,3-dihydro-1,3,3-trimethyl-2H-1,4-benzodiazepin-2-on vom Schmelzpunkt 188-189°.

Beispiel 12

Aus 4,5 g (0,011 Mol) 7-Amino-6-brom-5-(o-chlorphenyl)-1,3-dihydro-1,3,3-trimethyl-2H-1,4-benzodiazepin-2-on erhält man in Analogie zu den Angaben in Beispiel 4, über das [6-Brom-5-(o-chlorphenyl)-2,3-dihydro-1,3,3-trimethyl-2-oxo-1H-1,4-benzodiazepin-7-yl]isocyanat, 1-[6-Brom-5-(o-chlorphenyl)-2,3-dihydro-1,3,3-trimethyl-2-oxo-1H-1,4-benzodiazepin-7-yl]-3-(2-hydroxyäthyl)harnstoff vom Schmelzpunkt 214-218° (Aceton/Aether).

Beispiel 13

a) Unter Rühren versetzt man eine Lösung von 160 g (0,49 Mol) 5-(o-Fluorphenyl)-1,3-dihydro-3,3-dimethyl-7-nitro-2H-1,4-benzodiazepin-2-on in 400 ml konz. Salzsäure und 400 ml Ameisensäure mit einer Lösung von 46 g Chlorgas in 400 ml 1,2-Dichloräthan. Das aus zwei Phasen bestehende Reaktionsgemisch wird bei Raumtemperatur gerührt, wobei man nach 3 Tagen weitere 25 g Chlorgas in 200 ml 1,2-Dichloräthan und nach 5 Tagen noch 5 g Chlorgas in 40 ml 1,2-Dichloräthan zugibt. Nach 7 Tagen wird das Gemisch eingeengt, mit Eiswasser versetzt, mit wässrigem Ammoniak alkalisch gestellt und mit Methylenchlorid viermal extrahiert. Der organische Auszug wird eingeengt und mit 600 ml Isopropanol versetzt. Anschliessend entfernt man das restliche Methylenchlorid auf dem Dampfbad und lässt kristallisieren. Man erhält 9-Chlor-5-(o-fluorphenyl)-1,3-dihydro-3,3-dimethyl-7-nitro-2H-1,4-benzodiazepin-2-on vom Schmelzpunkt 174-175°.

b) Aus 120 g (0,33 Mol) 9-Chlor-5-(o-fluorphenyl)-1,3-dihydro-3,3-dimethyl-7-nitro-2H-1,4-benzodiazepin-2-on erhält man, in Analogie zu den Angaben in Absatz c) von Beispiel 1, 9-Chlor-5-(o-fluorphenyl)-1,3-dihydro-1,3,3-trimethyl-7-nitro-2H-1,4-benzodiazepin-2-on vom Schmelzpunkt 146-147° (Isopropanol/Methylenchlorid).

Beispiel 14

Aus 24 g (0,064 Mol) 9-Chlor-5-(o-fluorphenyl)-1,3-dihydro-1,3,3-trimethyl-7-nitro-2H-1,4-benzodiazepin-2-on erhält man, in Analogie zu den Angaben in Beispiel 2, 7-Amino-9-chlor-5-(o-fluorphenyl)-1,3-dihydro-1,3,3-trimethyl-2H-1,4-benzodiazepin-2-on vom Schmelzpunkt 237-238° (Isopropanol).

Beispiel 15

Aus 5,1 g (0,015 Mol) 7-Amino-5-(o-chlorphenyl)-1,3-dihydro-1,3,3-trimethyl-2H-1,4-benzodiazepin-2-on und Pyrrolidin erhält man in Analogie zu den Angaben in Beispiel 4, über das [5-(o-Chlorphenyl)2,3-dihydro-1,3,3-trimethyl-2-oxo-1H-1,4-benzodiazepin-7-yl]isocyanat, N-[5-(o-Chlorphenyl)-2,3-dihydro-1,3,3-trimethyl-2-oxo-1H-1,4-benzodiazepin-7-yl]-1-pyrrolidincarboxamid vom Schmelzpunkt 248-150° (Essigester/Aether).

Beispiel 16

Aus 25 g (0,073 Mol) 5-(o-Chlorphenyl)-1,3-dihydro-3,3-dimethyl-7-nitro-2H-1,4-benzodiazepin-2-on und Isopropylbromid erhält man in Analogie zu den Angaben in Absatz c) von Beispiel 1, jedoch bei einer Reaktionsdauer von 7 Tagen, 5-(o-Chlorphenyl)-1,3-dihydro-1-isopropyl-3,3-dimethyl-7-nitro-2H-1,4-benzodiazepin-2-on vom Schmelzpunkt 197-198° (Aether/n-Hexan).

Beispiel 17

Aus 1,7 g (0,005 Mol) 5-(o-Chlorphenyl)-1,3-dihydro-3,3-dimethyl-7-nitro-2H-1,4-benzodiazepin-2-on und Aethylbromid erhält man in Analogie zu den Angaben in Absatz c) von Beispiel 1, bei einer Reaktionsdauer von 36 Stunden, 5-(o-Chlorphenyl)-1-äthyl-1,3-dihydro-3,3-dimethyl-7-nitro-2H-1,4-benzodiazepin-2-on vom Schmelzpunkt 174° (Aether).

Beispiel 18

Aus 25 g (0,067 Mol) 5-(o-Chlorphenyl)-1-äthyl-1,3-dihydro-3,3-dimethyl-7-nitro-2H-1,4-benzodiazepin-2-on erhält man, in Analogie zu den Angaben in Beispiel 2, 7-Amino-5-(o-chlorphenyl)-1-äthyl-1,3-dihydro-3,3-dimethyl-2H-1,4-benzodiazepin-2-on vom Schmelzpunkt 178° (Aether).

Beispiel 19

Aus 25 g (0,073 Mol) 5-(o-Chlorphenyl)-1,3-dihydro-3,3-dimethyl-7-nitro-2H-1,4-benzodiazepin-2-on und n-Butylbromid erhält man in Analogie zu den Angaben in Absatz c) von Beispiel 1, bei einer Reaktionsdauer von 4 Tagen, 1-Butyl-5-(o-chlorphenyl)-1,3-dihydro-3,3-dimethyl-7-nitro-2H-1,4-benzodiazepin-2-on vom Schmelzpunkt 129° (Aether/n-Hexan).

Beispiel 20

Aus 25 g (0,073 Mol) 5-(o-Chlorphenyl)-1,3-dihydro-3,3-dimethyl-7-nitro-2H-1,4-benzodiazepin-2-on und 2-Bromäthanol erhält man in Analogie zu den Angaben in Absatz c) von Beispiel 1, bei einer Reaktionsdauer von 7 Tagen, 5-(o-Chlorphenyl)-1,3-dihydro-1-(2-hydroxyäthyl)-3,3-dimethyl-7-nitro-2H-1,4-benzodiazepin-2-on vom Schmelzpunkt 124-125° (Aether/n-Hexan).

Beispiel 21

Aus 25 g (0,073 Mol) 5-(o-Chlorphenyl)-1,3-dihydro-3,3-dimethyl-7-nitro-2H-1,4-benzodiazepin-2-on und 2-Diäthylamino-äthylchlorid erhält man in Analogie zu den Angaben in Absatz c) von Beispiel 1, bei einer Reaktionsdauer von 60 Stunden, 5-(o-Chlorphenyl)-1,3-dihydro-1-(2-diäthylamino-äthyl)-3,3-dimethyl-7-nitro-2H-1,4-benzodiazepin-2-on vom Schmelzpunkt 109° (Aether/n-Hexan).

Beispiel 22

Aus 12,5 g (0,032 Mol) 5-(o-Chlorphenyl)-1,3-dihydro-1-(2-hydroxyäthyl)-3,3-dimethyl-7-nitro-2H-1,4-benzodiazepin-2-on erhält man, in Analogie zu den Angaben in Beispiel 2, 7-Amino-5-(o-chlorphenyl)-1,3-dihydro-1-(2-hydroxyäthyl)-3,3-dimethyl-2H-1,4-benzodiazepin-2-on vom Schmelzpunkt 192-194° (Essigester/Aether).

Beispiel 23

Aus 6,5 g (0,018 Mol) 7-Amino-5-(o-chlorphenyl)-1,3-dihydro-1-(2-hydroxyäthyl)-3,3-dimethyl-2H-1,4-benzodiazepin-2-on erhält man in Analogie zu den Angaben in Absatz b) von Beispiel 4, über das [5-(o-Chlorphenyl)-2,3-dihydro-1-(2-hydroxyäthyl)-3,3-dimethyl-2-oxo-1H-1,4-benzodiazepin-7-yl]isocyanat, 1-[5-(o-Chlorphenyl)-2,3-dihydro-1-(2-hydroxyäthyl)-3,3-dimethyl-2-oxo-1H-1,4-benzodiazepin-7-yl]-3-(2-hydroxyäthyl)-harnstoff vom Schmelzpunkt 175-176° (Aethanol/Essigester).

Beispiel 24

Aus 10 g (0,022 Mol) 5-(o-Chlorphenyl)-1-(2-diäthylamino-äthyl)-1,3-dihydro-3,3-dimethyl-7-nitro-2H-1,4-benzodiazepin-2-on erhält man, in Analogie zu den Angaben in Beispiel 2, 7-Amino-5-(o-chlorphenyl)-1-(2-diäthylamino-äthyl)-1,3-dihydro-3,3-dimethyl-2H-1,4-benzodiazepin-2-on

vom Schmelzpunkt 70° (Aethanol/Wasser).

Beispiel 25

Aus 7,5 g (0,019 Mol) 5-(o-Chlorphenyl)-1,3-dihydro-1-isopropyl-3,3-dimethyl-7-nitro-2H-1,4-benzodiazepin-2-on erhält man, in Analogie zu den Angaben in Beispiel 2, 7-Amino-5-(o-chlorphenyl)-1,3-dihydro-1-isopropyl-3,3-dimethyl-2H-1,4-benzodiazepin vom Schmelzpunkt 169-170° (Aether/n-Hexan).

Beispiel 26

Aus 4 g (0,011 Mol) 7-Amino-5-(o-chlorphenyl)-1,3-dihydro-1-isopropyl-3,3-dimethyl-2H-1,4-benzodiazepin-2-on erhält man in Analogie zu den Angaben in Beispiel 4, über das [5-(o-Chlorphenyl)-2,3-dihydro-1-isopropyl-3,3-dimethyl-2-oxo-1H-1,4-benzodiazepin-7-yl]isocyanat, 1-[5-(o-Chlorphenyl)-2,3-dihydro-1-isopropyl-3,3-dimethyl-2-oxo-1H-1,4-benzodiazepin-7-yl]-3-(2-hydroxyäthyl)harnstoff vom Schmelzpunkt 243-244° (1,2-Dichloräthan/Methylenchlorid).

Beispiel 27

8 g (0,026 Mol) 7-Amino-5-(o-fluorphenyl)-1,3-dihydro-1,3,3-trimethyl-2H-1,4-benzodiazepin-2-on werden mit 5 g α-Aminoisobuttersäurechlorid-hydrochlorid in 30 ml Tetrahydrofuran 1 Stunde bei Raumtemperatur gerührt, anschliessend gibt man 8 g Kaliumcarbonat hinzu und nach weiteren 15 Minuten versetzt man mit Methylenchlorid und Wasser. Die organische Phase wird abgetrennt, die wässerige Phase viermal mit Methylenchlorid ausgeschüttelt. Die vereinigten organischen Auszüge werden getrocknet und eingedampft. Der Rückstand wird an 350 g Kieselgel mit Methylenchlorid/Essigester (1:1) und Essigester als Elutionsmittel gereinigt. Aus Essigester/Aether erhält man 2-Amino-N-[5-(o-fluorphenyl)-2,3-dihydro-1,3,3-trimethyl-2-oxo-1H-1,4-benzodiazepin-7-yl]-2-methylpropionamid vom Schmelzpunkt 250°.

Beispiel 28

2 g (0,005 Mol) 2-Amino-N-[5-(o-fluorphenyl)-2,3-dihydro-1,3,3-trimethyl-2-oxo-1H-1,4-benzodiazepin-7-yl]-2-methylpropionamid werden in 50 ml 1,2-Dichloräthan gelöst und zu 1 g Phosgen in 50 ml 1,2-Dichloräthan bei Raumtemperatur gegeben. Das Reaktionsgemisch wird 1/2 Stunde gerührt und anschliessend mit Triäthylamin basisch gestellt. Man versetzt mit Wasser und extrahiert mit Methylenchlorid; die organische Phase wird getrocknet und eingedampft. Der Rückstand ergibt aus Essigester/Aether 3-[5-(o-Fluorphenyl)-2,3-dihydro-1,3,3-trimethyl-2-oxo-1H-1,4-benzodiazepin-7-yl]-5,5-dimethylhydantoin vom Schmelzpunkt 286-287°.

Beispiel 29

Aus 10 g (0,028 Mol) 9-Chlor-5-(o-fluorphenyl)-1,3-dihydro-3,3-dimethyl-7-nitro-2H-1,4-benzodiazepin-2-on und n-Butylbromid erhält man in Analogie zu den Angaben in Absatz c) von Beispiel 1, bei einer Reaktionsdauer von 9 Tagen und Reinigen des Rohproduktes durch Chromatographie an Kieselgel (Elutionsmittel: Methylenchlorid), 1-Butyl-9-chlor-5-(o-fluorphenyl)-1,3-dihydro-3,3-dimethyl-7-nitro-2H-1,4-benzodiazepin-2-on vom Schmelzpunkt 125-127° (Hexan/Petroläther).

Beispiel 30

4,75 g (0,014 Mol) 7-Amino-9-chlor-5-(o-fluorphenyl)-1,3-dihydro-1,3,3-trimethyl-2H-1,4-benzodiazepin-2-on werden in 100 ml trockenem Methylenchlorid gelöst und mit 4,2 g N-Chlorsuccinimid versetzt. Das Reaktionsgemisch wird 40 Stunden am Rückfluss erhitzt, mit 25-proz. wässrigem Ammoniak versetzt und mehrmals mit Methylenchlorid

extrahiert. Nach Trocknen und Eindampfen der organischen Phase wird der Rückstand an 250 g Kieselgel mit Methylenchlorid/Essigester (20:1) als Elutionsmittel chromatographiert. Man erhält 7-Amino-6,8,9-trichlor-1,3-dihydro-5-(o-fluorphenyl)-1,3,3-trimethyl-2H-1,4-benzodiazepin-2-on vom Schmelzpunkt 165-166° (Aether/n-Hexan).

Beispiel 31

Aus 4,75 g (0,014 Mol) 7-Amino-9-chlor-5-(o-fluorphenyl)-1,3-dihydro-1,3,3-trimethyl-2H-1,4-benzodiazepin-2-on und 2,1 g (0,0016 Mol) N-Chlorsuccinimid erhält man, in Analogie zu den Angaben in Beispiel 30, 7-Amino-6,9-dichlor-5-(o-fluorphenyl)-1,3-dihydro-1,3,3-trimethyl-2H-1,4-benzodiazepin-2-on vom Schmelzpunkt 190-192° (Aether/n-Hexan).

Beispiel 32

Aus 4,75 g (0,014 Mol) 7-Amino-9-chlor-5-(o-fluorphenyl)-1,3-dihydro-1,3,3-trimethyl-2H-1,4-benzodiazepin-2-on erhält man, in Analogie zu den Angaben in Beispiel 30, 7-Amino-8,9-dichlor-5-(o-fluorphenyl)-1,3-dihydro-1,3,3-trimethyl-2H-1,4-benzodiazepin-2-on vom Schmelzpunkt 169-172° (Aether).

Beispiel 33

Aus 10 g (0,028 Mol) 9-Chlor-5-(o-fluorphenyl)-1,3-dihydro-3,3-dimethyl-7-nitro-2H-1,4-benzodiazepin-2-on und Aethylbromid erhält man in Analogie zu den Angaben in Absatz c) von Beispiel 1, bei einer Reaktionsdauer von 8 Tagen und chromatographieren des Rohproduktes an Kieselgel (Elutionsmittel: Methylenchlorid), 9-Chlor-1-äthyl-5-(o-fluorphenyl)-1,3-dihydro-3,3-dimethyl-7-nitro-2H-1,4-benzodiazepin-2-on vom Schmelzpunkt 117-120° (Aether/n-Hexan).

Beispiel 34

Aus 3,8 g (0,01 Mol) 9-Chlor-1-äthyl-5-(o-fluorphenyl)-1,3-dihydro-3,3-dimethyl-7-nitro-2H-1,4-benzodiazepin-2-on erhält man, in Analogie zu den Angaben in Beispiel 2, 7-Amino-9-chlor-1-äthyl-5-(o-fluorphenyl)-1,3-dihydro-3,3-dimethyl-2H-1,4-benzodiazepin-2-on vom Schmelzpunkt 156° (Aether/n-Hexan).

Beispiel 35

a) Aus 10 g (0,029 Mol) 5-(o-Chlorphenyl)-1,3-dihydro-3,3-dimethyl-7-nitro-2H-1,4-benzodiazepin-2-on erhält man, in Analogie zu den Angaben in Absatz a) von Beispiel 13, 9-Chlor-5-(o-chlorphenyl)-1,3-dihydro-3,3-dimethyl-7-nitro-2H-1,4-benzodiazepin-2-on vom Schmelzpunkt 105° (Aether/n-Hexan).

b) Aus 3,1 g (0,0082 Mol) 9-Chlor-5-(o-chlorphenyl)-1,3-dihydro-3,3-dimethyl7-nitro-2H-1,4-benzodiazepin-2-on erhält man in Analogie zu den Angaben in Absatz c) von Beispeil 1 9-Chlor-5-(o-chlorphenyl)-1,3-dihydro-1,3,3-trimethyl-7-nitro-2H-1,4-benzodiazepin-2-on vom Schmelzpunkt 121° (Aether/n-Hexan).

Beispiel 36

Aus 2,2 g (0,0056 Mol) 9-Chlor-5-(o-chlorphenyl)-1,3-dihydro-1,3,3-trimethyl-7-nitro-2H-1,4-benzodiazepin-2-on erhält man in Analogie zu den Angaben in Beispiel 2 7-Amino-9-chlor-5-(o-chlorphenyl)-1,3-dihydro-1,3,3-trimethyl-2H-1,4-benzodiazepin-2-on vom Schmelzpunkt 254° (Aether/n-Hexan).

Beispiel 37

Aus 4,5 g (0,011 Mol) 1-Butyl-9-chlor-5-(o-fluorphenyl)-1,3-dihydro-3,3-dimethyl-7-nitro-2H-1,4-benzodiazepin-2-on erhält man in Analogie zu den Angaben in Beispiel 2 7-Amino-1-butyl-9-chlor-5-(o-fluorphenyl)-1,3-dihydro-3,3-dimethyl-2H-1,4-benzodiazepin-2-on vom Schmelzpunkt 151° (Aether/n-Hexan).

Beispiel 38

346 mg (1 mMol) 7-Amino-9-chlor-5-(o-fluorphenyl)-1,3-dihydro-1,3,3-trimethyl-2H-1,4-benzodiazepin-2-on werden in 4 ml Ameisensäure und 4 ml Formalinlösung 1 Stunde am Rückfluss erhitzt. Das Reaktionsgemisch wird mit Eis, wässrigem Ammoniak und Methylenchlorid versetzt, und mehrmals mit Methylenchlorid extrahiert. Nach Trocknen und Eindampfen der organischen Phase wird der Rückstand an 10 g Kieselgel (Korngrösse 0,04-0,06 mm) unter Druck (0,2-0,4 atm. $N_2$) mit Methylenchlorid als Elutionsmittel chromatographiert. Man erhält 9-Chlor-7-(dimethylamino)-5-(o-fluorphenyl)-1,3-dihydro-1,3,3-trimethyl-2H-1,4-benzodiazepin-2-on vom Schmelzpunkt 227° (Aether/Petroläther).

Beispiel 39

a) 12 g (0,035 Mol) 7-Amino-9-chlor-5-(o-fluorphenyl)-1,3-dihydro-1,3,3-trimethyl-2H-1,4-benzodiazepin-2-on werden in 360 ml trockenem Tetrahydrofuran gelöst, mit 10 g gemahlenem Natriumbicarbonat und 8 ml Chlorameisensäurebenzylester versetzt und 3 1/2 Stunden bei Raumtemperatur gerührt. Man versetzt mit Essigester und Wasser, extrahiert die abgetrennte wässrige Phase zweimal mit Essigester, trocknet die vereinigten organischen Auszüge und dampft sie ein. Der Rückstand wird an 600 g Kieselgel mit Methylenchlorid/Essigester (20:1) als Elutionsmittel chromatographiert. Aus Aether erhält man Benzyl-9-chlor-5-(o-fluorphenyl)-2,3-dihydro-1,3,3-trimethyl-2-oxo-1H-1,4-benzodiazepin-7-carbamat vom Schmelzpunkt 212°.

b) 9,6 g (0,02 Mol) Benzyl-9-chlor-5-(o-fluorphenyl)-2,3-dihydro-1,3,3-trimethyl-2-oxo-1H-1,4-benzodiazepin-7-carbamat in 50 ml t-Butanol versetzt man mit 3 g Kalium-t-butanolat und rührt 1/2 Stunde bei 40°. Nach Zugabe von 3,5 ml n-Butyljodid rührt man 28 Tage bei Raumtemperatur, versetzt mit Methylenchlorid und 10-proz. Natriumbicarbonat-Lösung und schüttelt die abgetrennte wässrige Phase noch zweimal mit Methylenchlorid aus. Die vereinigten organischen Auszüge werden getrocknet und eingedampft. Der Rückstand wird an 300 g Kieselgel (Korngrösse 0,04-0,06 mm) unter Druck (0,2-0,4 atm., $N_2$) mit Methylenchlorid/Essigester (20:1) als Elutionsmittel chromatographiert. Das so erhaltene Benzyl-N-butyl-9-chlor-5-(o-fluorphenyl)-2,3-dihydro-1,3,3-trimethyl-2-oxo-1H-1,4-benzodiazepin-7-carbamat wird direkt weiterverarbeitet.

c) 6 g des obigen Produktes in 80 ml trockenem Aethanol werden mit 500 mg 5-proz. Pd-C und Wasserstoff, mit einer Natronkalkpatrone (um $CO_2$ zu eliminieren), bei Raumtemperatur gerührt. Nach 6 Stunden wird vom Katalysator abfiltriert und eingeengt. Der Rückstand wird an 200 g Kieselgel (Korngrösse 0,04-0,06 mm) unter Druck (0,2-0,4 atm., $N_2$) mit Methylenchlorid/Essigester(10:1) als Elutionsmittel chromatographiert. Man erhält 7-(Butylamino)-9-chlor-5-(o-fluorphenyl)-1,3-dihydro-1,3,3-trimethyl-2H-1,4-benzodiazepin-2-on vom Schmelzpunkt 113-115° (Hexan).

## Beispiel 40

a) Aus 9,6 g (0,02 Mol) Benzyl-9-chlor-5-(o-fluorphenyl)-2,3-dihydro-1,3,3-trimethyl-2-oxo-1H-1,4-benzodiazepin-7-carbamat erhält man, in Analogie zu den Angaben in Absatz b) von Beispiel 39, Benzyl-9-chlor-N-äthyl-5-(o-fluorphenyl)-2,3-dihydro-1,3,3-trimethyl-2-oxo-1H-1,4-benzodiazepin-7-carbamat vom Schmelzpunkt 125-127° (Hexan).

b) Aus 9,6 g (0,019 Mol) Benzyl-9-chlor-N-äthyl-5-(o-fluorphenyl)-2,3-dihydro-1,3,3-trimethyl-2-oxo-1H-1,4-benzodiazepin-7-carbamat erhält man, in Analogie zu den Angaben in Absatz c) von Beispiel 39, 9-Chlor-7-(äthylamino)-5-(o-fluorphenyl)-1,3-dihydro-1,3,3-trimethyl-2H-1,4-benzodiazepin-2-on vom Schmelzpunkt 150-151° (Aether/Petroläther).

Beispiel 41

a) 30 g (0,092 Mol) 5-(o-Fluorphenyl)-1,3-dihydro-3,3-dimethyl-7-nitro-2H-1,4-benzodiazepin-2-on werden in 600 ml komz. Salzsäure und 600 ml Ameisensäure gelöst, mit 49,5 g N-Bromsuccinimid versetzt und 48 Stunden bei Raumtemperatur gerührt. Anschliessend gibt man noch zusätzlich 33 g N-Bromsuccinimid hinzu und rührt 4 Tage bei Raumtemperatur. Die Lösung wird eingedampft und der Rückstand auf Eis und wässriges Ammoniak gegossen. Nach dreimaligem Ausschütteln der wässrigen Lösung mit Methylenchlorid, wird die organische Phase getrocknet und eingedampft. Der Rückstand wird an 2 kg Kieselgel mit Methylenchlorid und Methylenchlorid/Essigester (20:1) als Elutionsmittel chromatographiert. Man erhält 9-Brom-5-(o-fluorphenyl)-1,3-dihydro-3,3-dimethyl-7-nitro-2H-1,4-benzodiazepin-2-on vom Schmelzpunkt 164° (Aether/n-Hexan).

b) Aus 21,8 g (0,054 Mol) 9-Brom-5-(o-fluorphenyl)-1,3-dihydro-3,3-dimethyl-7-nitro-2H-1,4-benzodiazepin-2-on erhält man in Analogie zu den Angaben in Absatz c) von Beispiel 1 9-Brom-5-(o-fluorphenyl)-1,3-dihydro-1,3,3-trimethyl-7-nitro-2H-1,4-benzodiazepin-2-on vom Schmelzpunkt 180° (Aether/Petroläther).

Beispiel 42

Aus 16 g (0,038 Mol) 9-Brom-5-(o-fluorphenyl)-1,3-dihydro-1,3,3-trimethyl-7-nitro-2H-1,4-benzodiazepin-2-on erhält man, in Analogie zu den Angaben in Beispiel 2, 7-Amino-9-brom-5-(o-fluorphenyl)-1,3-dihydro-1,3,3-trimethyl-2H-1,4-benzodiazepin-2-on vom Schmelzpunkt 228° (Methylenchlorid/Petroläther).

Beispiel 43

Aus 32 g (0,088 Mol) 9-Chlor-5-(o-fluorphenyl)-1,3-dihydro-3,3-dimethyl-7-nitro-2H-1,4-benzodiazepin-2-on und Propylbromid erhält man in Analogie zu den Angaben in Absatz c) von Beispiel 1, bei einer Reaktionsdauer von 3 Tagen, 9-Chlor-5-(o-fluorphenyl)-1,3-dihydro-3,3-dimethyl-7-nitro-1-propyl-2H-1,4-benzodiazepin-2-on vom Schmelzpunkt 142-143° (Aether/n-Hexan).

Beispiel 44

Aus 15 g (0,037 Mol) 9-Chlor-5-(o-fluorphenyl)-1,3-dihydro-3,3-dimethyl-7-nitro-1-propyl-2H-1,4-benzodiazepin-2-on erhält man, in Analogie zu den Angaben in Beispiel 2, 7-Amino-9-chlor-5-(o-fluorphenyl)-1,3-dihydro-3,3-dimethyl-1-propyl-2H-1,4-benzodiazepin-2-on vom Schmelzpunkt 178-179° (Essigester/Aether/n-Hexan).

Beispiel 45

Eine Lösung von 21,5 g (0,0691 Mol) 7-Amino-5-(o-fluorphenyl)-1,3-dihydro-1,3,3-trimethyl-2H-1,4-benzodiazepin-2-on und 25,8 g (0,145 Mol) N-Bromsuccinimid in 400 ml Methylenchlorid wird während 78 Stunden bei Raumtemperatur unter Argon gerührt und anschliessend auf 500 ml 2N Natriumcarbonat-Lösung gegossen. Die organische Phase wird abgetrennt und die wässrige Phase nochmals mit Methylenchlorid extrahiert. Die vereinigten organischen Auszüge werden mit Wasser gewaschen, getrocknet und eingedampft. Den Rückstand chromatographiert man an 500 g Kieselgel mit Chloroform als Elutionsmittel. Man erhält 7-Amino-6,8-dibrom-5-(o-fluorphenyl)-1,3-dihydro-1,3,3-trimethyl-2H-1,4-benzodiazepin-2-on vom Schmelzpunkt 202°.

Beispiel 46

Aus 20 g (0,061 Mol) 7-Amino-5-(o-chlorphenyl)-1,3-dihydro-1,3,3-trimethyl-2H-1,4-benzodiazepin-2-on erhält man, in Analogie zu den Angaben von Beispiel 45, 7-Amino-6,8-dibrom-5-(o-chlorphenyl)-1,3-dihydro-1,3,3-trimethyl-2H-1,4-benzodiazepin-2-on vom Schmelzpunkt 110° (Methylenchlorid/Cyclohexan).

Beispiel 47

Aus 20 g (0,0642 Mol) 7-Amino-5-(o-fluorphenyl)-1,3-dihydro-1,3,3-trimethyl-2H-1,4-benzodiazepin-2-on und 18 g (0,134 Mol) N-Chlorsuccinimid erhält man, in Analogie zu den Angaben in Beispiel 45, 7-Amino-6,8-dichlor-5-(o-fluorphenyl)-1,3-dihydro-1,3,3-trimethyl-2H-1,4-benzodiazepin-2-on vom Schmelzpunkt 208° (Methylenchlorid/Cyclohexan).

Beispiel 48

Aus 20 g (0,061 Mol) 7-Amino-5-(o-chlorphenyl)-1,3-dihydro-1,3,3-trimethyl-2H-1,4-benzodiazepin-2-on erhält man, in Analogie zu den Angaben in Beispiel 45, 7-Amino-6,8-dichlor-5-(o-chlorphenyl)-1,3-dihydro-1,3,3-trimethyl-2H-1,4-benzodiazepin-2-on vom Schmelzpunkt 105° (Methylenchlorid/Cyclohexan).

Beispiel 49

a) Eine Mischung aus 5 g (0,014 Mol) 2-Amino-2'-(o-fluorphenyl)-2-methyl-4'-nitro-propionanilid, 4 g Kaliumcarbonat (gemahlen) und 2,5 ml Chlorameisensäurebenzylester in 40 ml Tetrahydrofuran wird über Nacht bei Raumtemperatur gerührt und nach Abfiltrieren vom Kaliumcarbonat eingeengt. Der Rückstand wird an einer 250 g Kieselgel (Korngrösse 0,04-0,06 mm) enthaltenden Drucksäule unter 0,2-0,4 atm. $N_2$ mit Methylenchlorid als Elutionsmittel chromatographiert. Man erhält Benzyl-[1-[[2-(o-fluorbenzoyl)-4-nitrophenyl]carbamoyl]-1-methyl-äthyl]carbamat vom Schmelzpunkt 153-155° (n-Hexan).

b) Eine Lösung von 500 mg Benzyl-[1-[[2-(o-fluorbenzoyl)-4-nitrophenyl]-carbamoyl-1-methyl-äthyl]carbamat (1,043 Mol) und 150 mg Kalium-t-butanolat in 20 ml t-Butanol wird mit 0,15 ml Methyljodid versetzt und während 2,5 Tagen bei Raumtemperatur gerührt. Das Reaktionsgemisch wird mit Eisessig gepuffert und eingeengt. Der Rückstand wird zwischen Methylenchlorid und 10-proz. wässriger Natriumbicarbonat-Lösung verteilt. Nach Trocknen der organischen Phase über Natriumsulfat und Einengen wird der Rückstand an einer 10 g Kieselgel (Korngrösse 0,04-0,06 mm) enthaltenden Drucksäule unter 0,2-0,4 atm. $N_2$ mit Methylenchlorid/Essigester (20:1) als Elutionsmittel chromatographiert. Man erhält Benzyl-[1-[[2-(o-fluorbenzoyl)-4-nitrophenyl]-methylcarbamoyl]-1-methyl-äthyl]carbamat vom Schmelzpunkt 168-169° (Aether/n-Hexan).

c) Eine Lösung von 20 mg (0,04 Mol) Benzyl-[1-[[2-(o-fluorbenzoyl)-4-nitrophenyl]-methylcarbamoyl-1-methyl-äthyl]carbamat in 0,5 ml Bromwasserstoff/Eisessig (30-proz) wird während 15 Minuten stehengelassen, anschliessend mit einer 10-proz. Natriumbicarbonat-Lösung versetzt und mit Methylenchlorid mehrmals ausgeschüttelt. Die organische Phase wird über Natriumsulfat getrocknet und eingeengt, worauf der Rückstand an einer 2 g Kieselgel (Korngrösse 0,04-0,06 mm) enthaltenden Drucksäule unter 0,2-0,4 atm. $N_2$ mit Methylenchlorid als Elutionsmittel chromatographiert wird. Durch Kristallisation aus Aether/Petroläther erhält man 5-(o-Fluorphenyl)-1,3-dihydro-1,3,3-trimethyl-7-nitro-2H-1,4-benzodiazepin-2-on vom Schmelzpunkt 127-129°.

## Beispiel A

7-Amino-9-chlor-5-(o-fluorphenyl)-1,3-dihydro-1,3,3-trimethyl-2H-1,4-benzodiazepin-2-on kann wie folgt als Wirkstoff zur Herstellung von pharmazeutischen Präparaten verwendet werden:

a) Tabletten

| | 1 Tablette enthält |
|---|---|
| Wirkstoff | 200 mg |
| mikrokristalline Cellulose | 155 mg |
| Maisstärke | 25 mg |
| Talk | 25 mg |
| Hydroxypropylmethylcellulose | 20 mg |
| | 425 mg |

Der Wirkstoff wird mit der Hälfte der mikrokristallinen Cellulose gemischt und mit einer 10-proz. Lösung von Hydroxypropylmethylcellulose in einem Gemisch von Isopropanol und Methylenchlorid granuliert. Das Granulat wird getrocknet, gesiebt und mit dem Rest der Hilfsstoffe gemischt. Alsdann wird es auf einer Presse zu biplanen Tabletten von 12 mm Durchmesser mit Kreuzbruchrille verpresst.

b) Kapseln

| | 1 Kapsel enthält |
|---|---|
| Wirkstoff | 100,0 mg |
| Maisstärke | 20,0 mg |
| Milchzucker | 95,0 mg |
| Talk | 4,5 mg |
| Magnesiumstearat | 0,5 mg |
| | 220,0 mg |

Der Wirkstoff wird mit den Hilsstoffen gemischt und gesiebt.Nach erneutem Mischen wird die erhaltene Kapselfüllmasse auf einer vollautomatischen Kapselabfüllmaschine in Gelatinesteckkapseln geeigneter Grösse abgefüllt.

## Patentansprüche

1. Benzodiazepin-Derivate der allgemeinen Formel

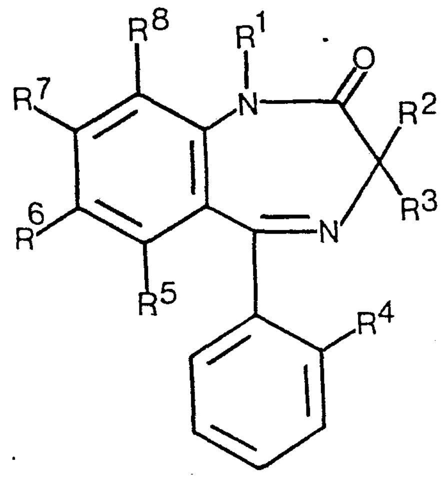

I

worin $R^1$ niederes Alkyl, niederes Hydroxyalkyl oder niederes Dialkylaminoalkyl, $R^2$ und $R^3$ je niederes Alkyl, $R^4$ Halogen, $R^5$, $R^7$ und $R^8$ je Wasserstoff oder Halogen und $R^6$ Nitro, Amino, niederes Alkylamino, niederes Dialkylamino oder einen Rest der Formel $H_2N-C(CH_3)_2-CO-NH-$, $R^9R^{10}N-CO-NH-$ oder

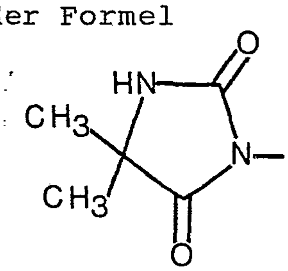

bedeuten und entweder $R^9$ Wasserstoff oder niederes Alkyl und $R^{10}$ niederes Alkyl oder niederes Hydroxyalkyl bedeuten oder $R^9$ und $R^{10}$ zusammen mit dem Stickstoffatom einen 3- bis 7-gliedrigen Heterocyclus bedeuten, der - falls er mindestens 5-gliedrig ist - ein Sauerstoff- oder Schwefelatom oder einen Rest der Formel $>N-R$ als Ringglied enthalten kann, wobei R Wasserstoff oder niederes Alkyl bedeutet,
und pharmazeutisch annehmbare Säureadditionssalze davon.

2. Verbindungen gemäss Anspruch 1, dadurch gekennzeichnet, dass $R^1$ Methyl bedeutet.

3. Verbindungen gemäss Anspruch 1 oder 2, dadurch gekennzeichnet, dass $R^2$ und $R^3$ beide Methyl bedeuten.

4. Verbindungen gemäss einem der Ansprüche 1 bis 3, dadurch gekennzeichnet, dass $R^4$ Fluor oder Chlor bedeutet.

5. Verbindungen gemäss einem der Ansprüche 1 bis 4, dadurch gekennzeichnet, dass $R^5$ Wasserstoff, Chlor oder Brom bedeutet.

6. Verbindungen gemäss einem der Ansprüche 1 bis 5, dadurch gekennzeichnet, dass $R^7$ Wasserstoff, Chlor oder Brom bedeutet.

7. Verbindungen gemäss einem der Ansprüche 1 bis 6, dadurch gekennzeichnet, dass $R^8$ Wasserstoff oder Chlor bedeutet.

8. 7-Amino-9-chlor-5-(o-fluorphenyl)-1,3-dihydro-1,3,3-trimethyl-2H-1,4-benzodiazepin-2-on.

9. 7-Amino-6,8-dibrom-5-(o-fluorphenyl)-1,3-dihydro-1,3,3-trimethyl-2H-1,4-benzodiazepin-2-on.

10. 7-Amino-6,8-dibrom-5-(o-chlorphenyl)-1,3-dihydro-1,3,3-trimethyl-2H-1,4-benzodiazepin-2-on.

11. 7-Amino-6,8-dichlor-5-(o-chlorphenyl)-1,3-dihydro-1,3,3-trimethyl-2H-1,4-benzodiazepin-2-on.

12. 1-[6-Brom-5-(o-chlorphenyl)-2,3-dihydro-1,3,3-trimethyl-2-oxo-1H-1,4-benzodiazepin-7-yl]-3-(2-hydroxyäthyl)-harnstoff, 7-Amino-9-chlor-5-(o-chlorphenyl)-1,3-dihydro-1,3,3-trimethyl-2H-1,4-benzodiazepin-2-on, 1-[5-(o-Chlorphenyl)-2,3-dihydro-1,3,3-trimethyl-2-oxo-1H-1,4-benzodiazepin-7-yl]-3-(2-hydroxyäthyl)harnstoff, 7-Amino-6,8,9-trichlor-1,3-dihydro-5-(o-fluorphenyl)-1,3,3-trimethyl-2H-1,4-benzodiazepin-2-on, 7-Amino-8,9-dichlor-5-(o-fluorphenyl)-1,3-dihydro-1,3,3-trimethyl-2H-1,4-benzodiazepin-2-on, 7-Amino-6-chlor-5-(o-fluorphenyl)-1,3-dihydro-1,3,3-trimethyl-2H-1,4-benzodiazepin-2-on und 2-Amino-N-[5-(o-fluorphenyl)-2,3-dihydro-1,3,3-trimethyl-2-oxo-1H-1,4-benzodiazepin-7-yl]-2-methylpropionamid.

13. Verbindungen der allgemeinen Formel

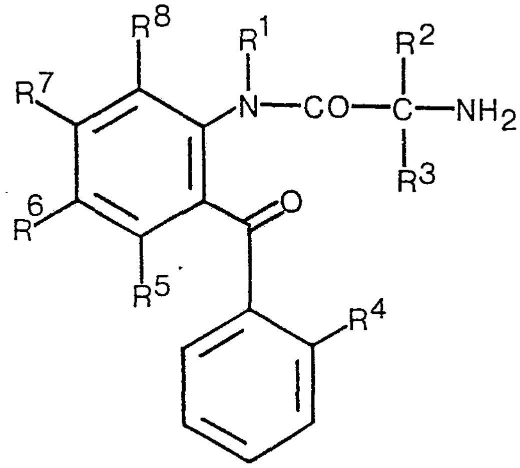

II

worin $R^1$ niederes Alkyl, niederes Hydroxyalkyl oder niederes Dialkylaminoalkyl, $R^2$ und $R^3$ je niederes Alkyl, $R^4$ Halogen, $R^5$, $R^7$ und $R^8$ je Wasserstoff oder Halogen und $R^6$ Nitro, Amino, niederes Alkylamino, niederes Dialkylamino oder einen Rest der Formel $H_2N-C(CH_3)_2-CO-NH-$, $R^9 R^{10}N-CO-NH-$ oder 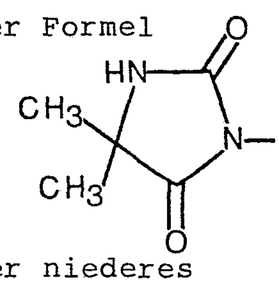

bedeuten und entweder $R^9$ Wasserstoff oder niederes Alkyl und $R^{10}$ niederes Alkyl oder niederes Hydroxyalkyl bedeuten oder $R^9$ und $R^{10}$ zusammen mit dem Stickstoffatom einen 3- bis 7-gliedrigen Heterocyclus bedeuten, der - falls er mindestens 5-gliedrig ist - ein Sauerstoff- oder Schwefelatom oder einen Rest der Formel $>N-R$ als Ringglied enthalten kann, wobei

R Wasserstoff oder niederes Alkyl bedeutet.

14. Benzodiazepin-Derivate der allgemeinen Formel

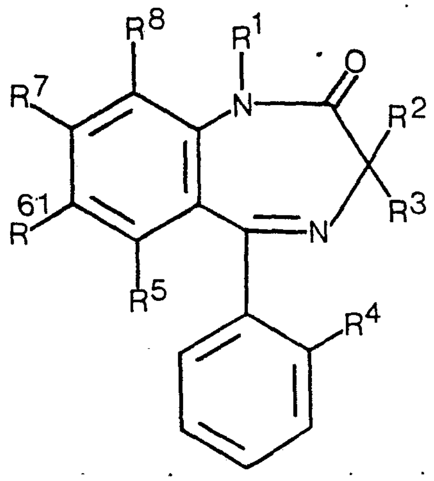

III

worin $R^1$ niederes Alkyl, niederes Hydroxyalkyl oder niederes Dialkylaminoalkyl, $R^2$ und $R^3$ je niederes Alkyl, $R^4$ Halogen, $R^5$, $R^7$ und $R^8$ je Wasserstoff oder Halogen und $R^{61}$ geschütztes niederes Alkylamino oder einen Rest der Formel ZZ'N-, Z-NH-$C(CH_3)_2$-CO-NH- oder $R^{91}R^{101}$N-CO-NH- bedeuten und entweder $R^{91}$ Wasserstoff oder niederes Alkyl und $R^{101}$ einen Rest der Formel -A-O-Z bedeuten oder $R^{91}$ eine Schutzgruppe und $R^{101}$ niederes Alkyl oder einen Rest der Formel -A-O-Z bedeuten, oder $R^{91}$ und $R^{101}$ zusammen mit dem Stickstoffatom einen 5- bis 7-gliedrigen Heterocyclus bedeuten, der einen Rest der Formel $>$N-$R^{01}$ als Ringglied enthält, wobei $R^{01}$ eine Schutzgruppe bedeutet, und A niederes Alkylen und Z und Z' je eine Schutzgruppe bedeuten, wobei Z zusammen mit Z' eine Schutzgruppe bedeuten kann.

15. Benzodiazepin-Derivate der allgemeinen Formel

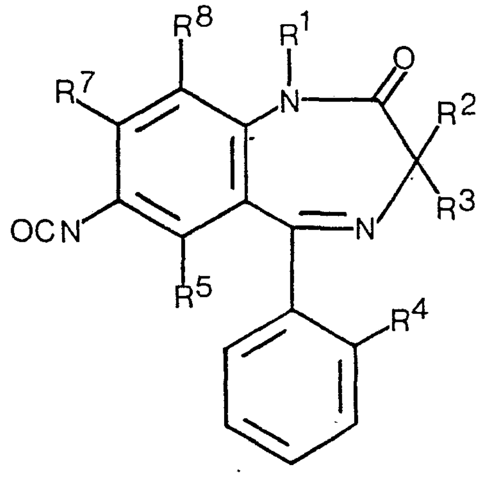

VI

worin $R^1$ niederes Alkyl, niederes Hydroxyalkyl oder niederes Dialkylaminoalkyl, $R^2$ und $R^3$ je niederes Alkyl, $R^4$ Halogen und $R^5$, $R^7$ und $R^8$ je Wasserstoff oder Halogen bedeuten.

16. Verbindungen gemäss einem der Ansprüche 1 bis 12 als pharmazeutische Wirkstoffe.

17. Verbindungen gemäss einem der Ansprüche 1 bis 12 als aldosteronantagonistische Wirkstoffe.

18. Verfahren zur Herstellung von Benzodiazepin-Derivaten gemäss einem der Ansprüche 1 bis 12, dadurch gekennzeichnet, dass man

a) eine Verbindung der allgemeinen Formel

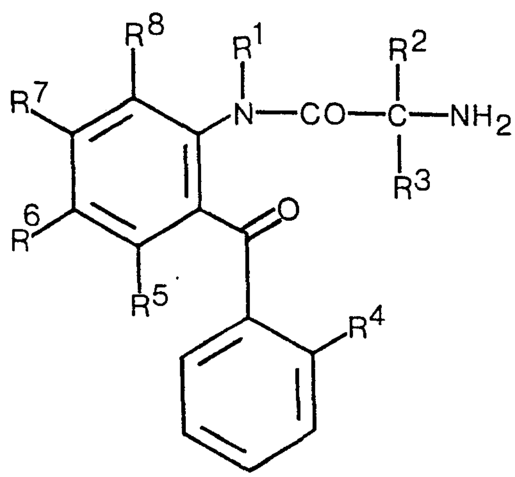

II

worin $R^1$, $R^2$, $R^3$, $R^4$, $R^5$, $R^6$, $R^7$ und $R^8$ die in Anspruch 1 angegebene Bedeutung besitzen,

cyclisiert, oder

b) aus einer Verbindung der allgemeinen Formel

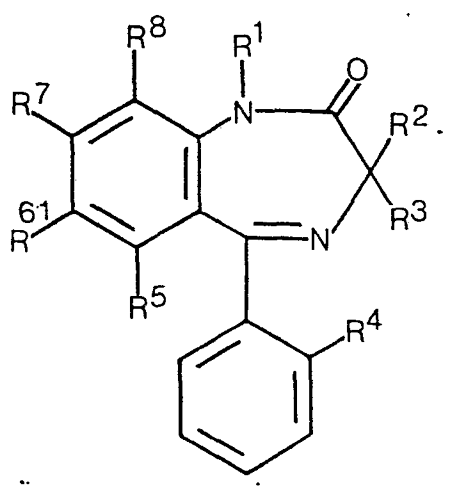

III

worin $R^1$, $R^2$, $R^3$, $R^4$, $R^5$, $R^7$ und $R^8$ die in Anspruch 1 angegebene Bedeutung besitzen und $R^{61}$ geschütztes niederes Alkylamino oder einen Rest der Formel ZZ'N-, $Z-NH-C(CH_3)_2-CO-NH-$ oder $R^{91}R^{101}N-CO-NH-$ bedeuten und entweder $R^{91}$ Wasserstoff oder niederes Alkyl und $R^{101}$ einen Rest der Formel -A-O-Z bedeuten oder $R^{91}$ eine Schutzgruppe und $R^{101}$ niederes Alkyl oder einen Rest der Formel -A-O-Z bedeuten, oder $R^{91}$ und $R^{101}$ zusammen mit dem Stickstoffatom einen 5- bis 7-gliedrigen Heterocyclus bedeuten, der einen Rest der Formel $>N-R^{01}$ als Ringglied enthält, wobei $R^{01}$ eine Schutzgruppe bedeutet, und A niederes Alkylen und Z und Z' je eine Schutzgruppe bedeuten, wobei Z zusammen mit Z' eine Schutzgruppe bedeuten kann, die Schutzgruppe(n) abspaltet, oder

c) ein 3,3-Dialkylbenzodiazepin-Derivat der allgemeinen Formel

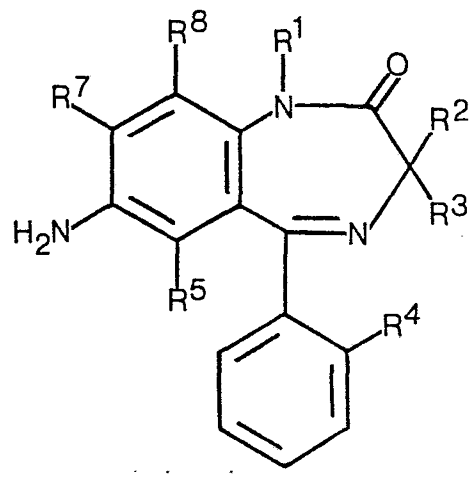

Ia

worin $R^1$, $R^2$, $R^3$, $R^4$, $R^5$, $R^7$ und $R^8$ die in Anspruch 1 angegebene Bedeutung besitzen,

mit einem Halogenid der allgemeinen Formel

$$R^{92}R^{102}N-CO-X \qquad IV$$

worin X Halogen bedeutet und entweder $R^{92}$ und

$R^{102}$ je niederes Alkyl bedeuten oder $R^{92}$ und $R^{102}$ zusammen mit dem Stickstoffatom einen 3- bis 7-gliedrigen Heterocyclus bedeuten, der - falls er mindestens 5-gliedrig ist - ein Sauerstoff- oder Schwefelatom oder einen Rest der Formel $>N-R^{02}$ enthalten kann, wobei $R^{02}$ niederes Alkyl bedeutet,

umsetzt, oder

d) ein 3,3-Dialkylbenzodiazepin-Derivat der obigen allgemeinen Formel Ia mit einem Isocyanat der allgemeinen Formel

$$R^{93}-NCO \qquad V$$

worin $R^{93}$ niederes Alkyl bedeutet,

umsetzt, oder

e) eine Verbindung der allgemeinen Formel

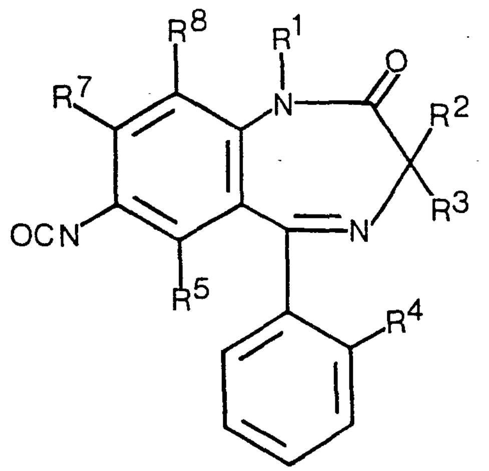

VI

worin $R^1$, $R^2$, $R^3$, $R^4$, $R^5$, $R^7$ und $R^8$ die in Anspruch 1 angegebene Bedeutung besitzen,

mit einer Aminoverbindung der allgemeinen Formel

$$R^9R^{10}NH \qquad VII$$

worin $R^9$ und $R^{10}$ die in Anspruch 1 angegebene Bedeutung besitzen,

umsetzt, oder

f) eine Verbindung der allgemeinen Formel

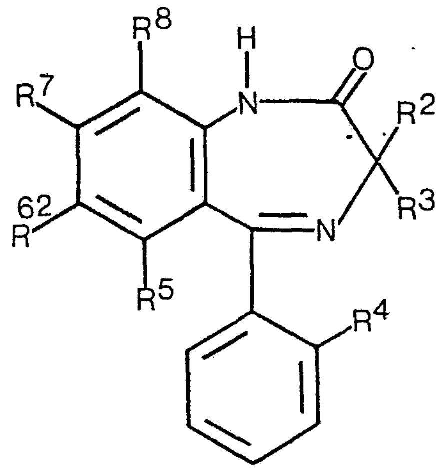

VIII

worin $R^2$, $R^3$, $R^4$, $R^5$, $R^7$ und $R^8$ die in Anspruch 1 angegebene Bedeutung besitzen und $R^{62}$ Nitro oder niederes Dialkylamino bedeutet,

mit einem den Rest $R^1$ liefernden Alkylierungsmittel behandelt, oder

g) ein 3,3-Dialkylbenzodiazepin-Derivat der allgemeinen Formel

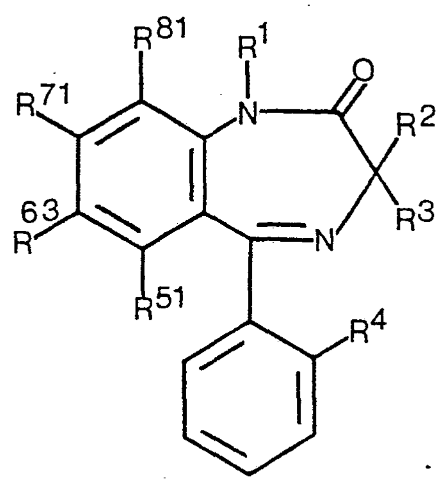

Ib

worin $R^1$, $R^2$, $R^3$ und $R^4$ die in Anspruch 1 angegebene Bedeutung besitzen, und entweder $R^{63}$ Nitro und $R^{51}$, $R^{71}$ und $R^{81}$ alle Wasserstoff bedeuten oder $R^{63}$ Amino oder niederes Alkylamino und $R^{81}$ Wasserstoff oder Halogen bedeuten, und einer der Reste $R^{51}$ und $R^{71}$ Wasserstoff

und der andere Wasserstoff oder Halogen bedeutet, halogeniert, oder

h) in einem 3,3-Dialkylbenzodiazepin-Derivat der obigen allgemeinen Formel Ia die primäre Aminogruppe in die Nitrogruppe überführt, oder

i) in einem 3,3-Dialkylbenzodiazepin-Derivat der allgemeinen Formel

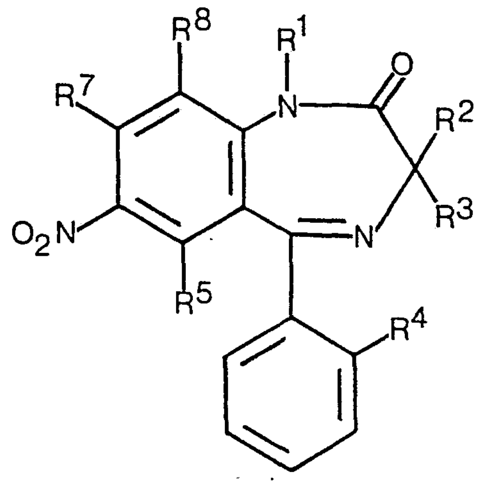

Ic

worin $R^1$, $R^2$, $R^3$, $R^4$, $R^5$, $R^7$ und $R^8$ die in Anspruch 1 angegebene Bedeutung besitzen,

die Nitrogruppe zur Aminogruppe reduziert, oder

j) in einem 3,3-Dialkylbenzodiazepin-Derivat der obigen allgemeinen Formel Ia die primäre Aminogruppe mono- oder dialkyliert, oder

k) in einem Dialkylbenzodiazepin-Derivat der allgemeinen Formel

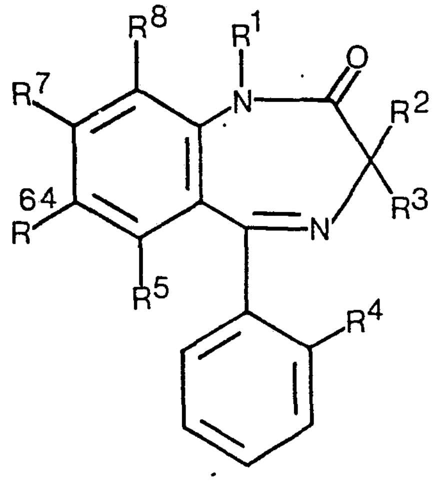

Id

worin $R^1$, $R^2$, $R^3$, $R^4$, $R^5$, $R^7$ und $R^8$ die in Anspruch 1 angegebene Bedeutung besitzen und $R^{64}$ niederes Alkylamino bedeutet,

die sekundäre Aminogruppe alkyliert, oder

l) ein 3,3-Dialkylbenzodiazepin-Derivat der obigen allgemeinen Formel Ia mit einem Dihalogenid der allgemeinen Formel

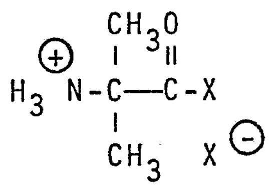

IX

worin X obige Bedeutung besitzt,

umsetzt, oder

m) ein 3,3-Dialkylbenzodiazepin-Derivat der allgemeinen Formel

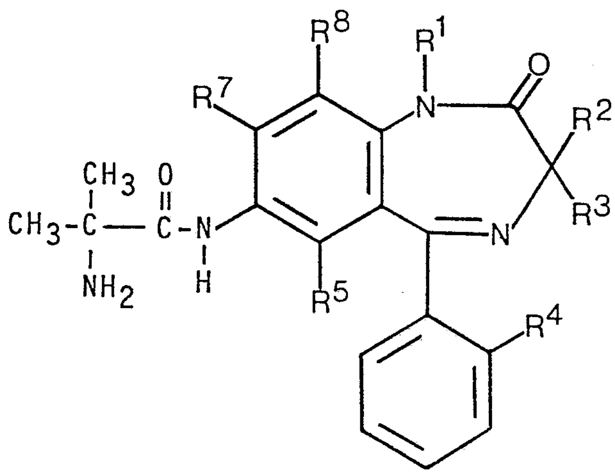

Ie

worin $R^1$, $R^2$, $R^3$, $R^4$, $R^5$, $R^7$ und $R^8$ die in Anspruch 1 angegebene Bedeutung besitzen,

mit Phosgen behandelt, oder

n) in einem 3,3-Dialkylbenzodiazepin-Derivat der allgemeinen Formel

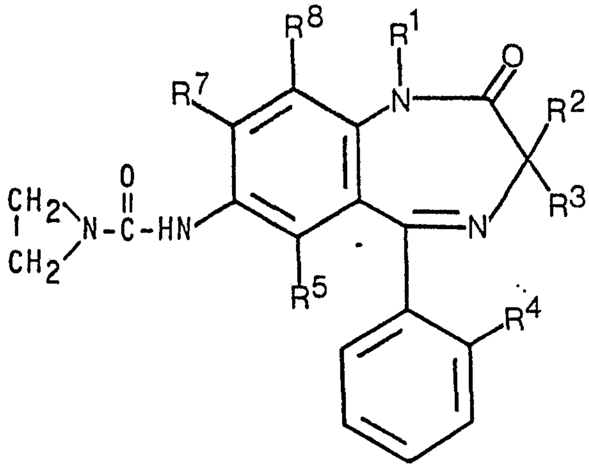

If

worin $R^1$, $R^2$, $R^3$, $R^4$, $R^5$, $R^7$ und $R^8$ die in Anspruch 1 angegebene Bedeutung besitzen,

den Aziridinring hydrolytisch öffnet, oder

o) ein 3,3-Dialkylbenzodiazepin-Derivat der allgemeinen Formel I in ein pharmazeutisch annehmbares Säureadditionssalz überführt.

19. Arzneimittel, enthaltend eine Verbindung gemäss einem der Ansprüche 1 bis 12.

20. Aldosteronantagonistische Arzneimittel, enthaltend eine Verbindung gemäss einem der Ansprüche 1 bis 12.

Patentansprüche
"für OESTERREICH"

1. Verfahren zur Herstellung von Benzodiazepin-Derivaten der allgemeinen Formel

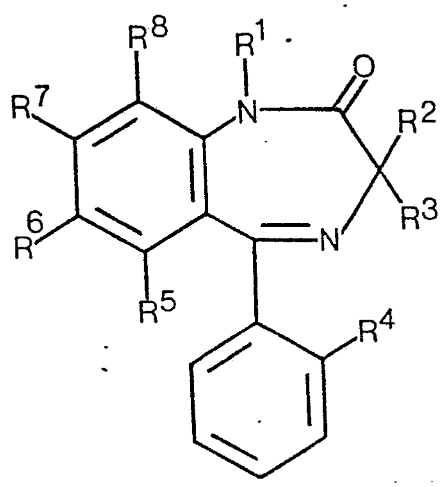

I

worin $R^1$ niederes Alkyl, niederes Hydroxyalkyl oder niederes Dialkylaminoalkyl, $R^2$ und $R^3$ je niederes Alkyl, $R^4$ Halogen, $R^5$, $R^7$ und $R^8$ je Wasserstoff oder Halogen und $R^6$ Nitro, Amino, niederes Alkylamino, niederes Dialkylamino oder einen Rest der Formel $H_2N-C(CH_3)_2-CO-NH-$, $R^9R^{10}N-CO-NH-$ oder

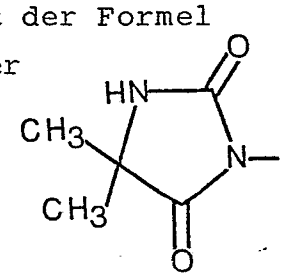

bedeuten und entweder $R^9$ Wasserstoff oder niederes Alkyl und $R^{10}$ niederes Alkyl oder niederes Hydroxyalkyl bedeuten oder $R^9$ und $R^{10}$ zusammen mit dem Stickstoffatom einen 3- bis 7-gliedrigen Heterocyclus bedeuten, der - falls er mindestens 5-gliedrig ist - ein Sauerstoff- oder Schwefelatom oder einen Rest der Formel $\rangle$N-R als Ringglied enthalten kann, wobei R Wasserstoff oder niederes Alkyl bedeutet,

und von pharmazeutisch annehmbaren Säureadditionssalzen davon, dadurch gekennzeichnet, dass man

a) eine Verbindung der allgemeinen Formel

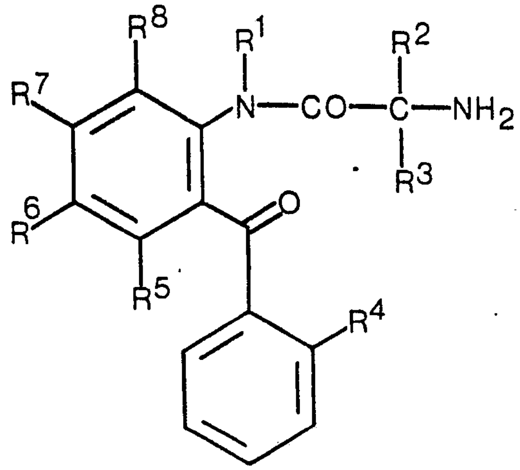

II

worin $R^1$, $R^2$, $R^3$, $R^4$, $R^5$, $R^6$, $R^7$ und $R^8$ die in obige Bedeutung besitzen,

cyclisiert, oder

b) aus einer Verbindung der allgemeinen Formel

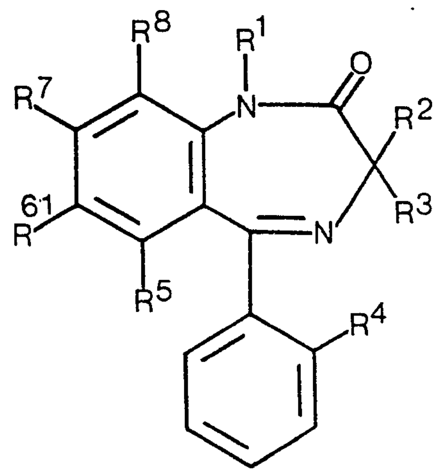

III

worin $R^1$, $R^2$, $R^3$, $R^4$, $R^5$, $R^7$ und $R^8$ obige Bedeutung besitzen und $R^{61}$ geschütztes niederes Alkylamino oder einen Rest der Formel ZZ'N-, Z-NH-C($CH_3$)$_2$-CO-NH- oder $R^{91}R^{101}$N-CO-NH- bedeuten und entweder $R^{91}$ Wasserstoff oder niederes Alkyl und $R^{101}$ einen Rest der Formel -A-O-Z bedeuten oder $R^{91}$ eine Schutzgruppe und $R^{101}$ niederes Alkyl oder einen Rest der Formel -A-O-Z bedeuten, oder $R^{91}$ und $R^{101}$ zusammen mit dem Stickstoffatom einen 5- bis 7-gliedrigen Heterocyclus bedeuten, der einen Rest der Formel >N-$R^{01}$ als Ring-

glied enthält, wobei $R^{01}$ eine Schutzgruppe bedeutet, und A niederes Alkylen und Z und Z' je eine Schutzgruppe bedeuten, wobei Z zusammen mit Z' eine Schutzgruppe bedeuten kann, die Schutzgruppe(n) abspaltet, oder

c) ein 3,3-Dialkylbenzodiazepin-Derivat der allgemeinen Formel

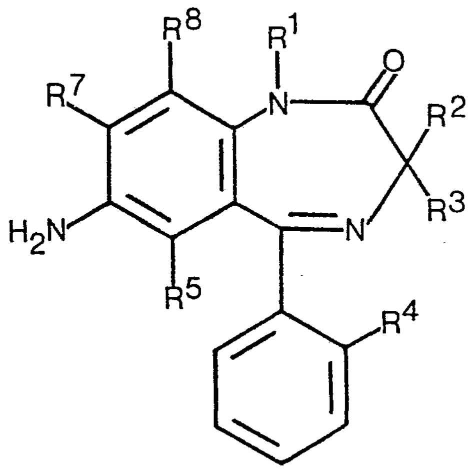

Ia

worin $R^1$, $R^2$, $R^3$, $R^4$, $R^5$, $R^7$ und $R^8$ obige Bedeutung besitzen,

mit einem Halogenid der allgemeinen Formel

$$R^{92}R^{102}N\text{-}CO\text{-}X \qquad IV$$

worin X Halogen bedeutet und entweder $R^{92}$ und $R^{102}$ je niederes Alkyl bedeuten oder $R^{92}$ und $R^{102}$ zusammen mit dem Stickstoffatom einen 3- bis 7-gliedrigen Heterocyclus bedeuten, der - falls er mindestens 5-gliedrig ist - ein Sauerstoff- oder Schwefelatom oder einen Rest der Formel $>N\text{-}R^{02}$ enthalten kann, wobei $R^{02}$ niederes Alkyl bedeutet,

umsetzt, oder

d) ein 3,3-Dialkylbenzodiazepin-Derivat der obigen allgemeinen Formel Ia mit einem Isocyanat der allgemeinen Formel

$R^{93}$-NCO V

worin $R^{93}$ niederes Alkyl bedeutet,
umsetzt, oder

e) eine Verbindung der allgemeinen Formel

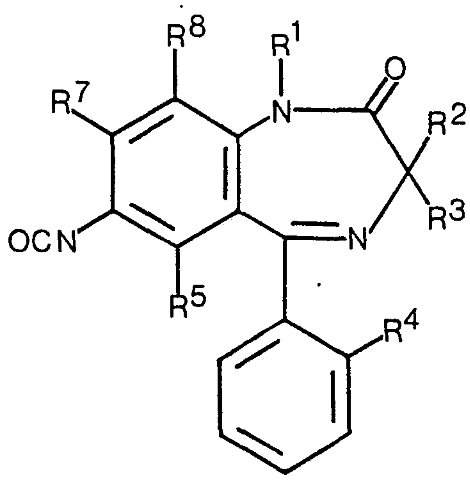

VI

worin $R^1$, $R^2$, $R^3$, $R^4$, $R^5$, $R^7$ und $R^8$ obige Bedeutung besitzen,
mit einer Aminoverbindung der allgemeinen Formel

$R^9R^{10}NH$ VII

worin $R^9$ und $R^{10}$ obige Bedeutung besitzen,
umsetzt, oder

f) eine Verbindung der allgemeinen Formel

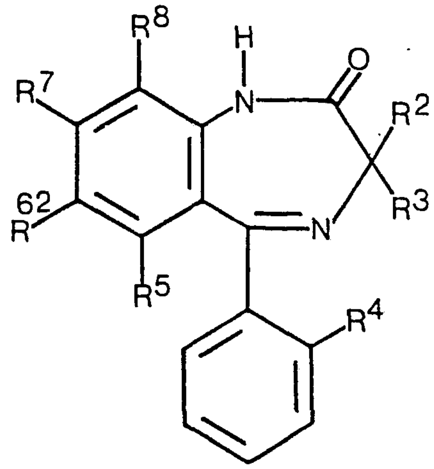

VIII

worin $R^2$, $R^3$, $R^4$, $R^5$, $R^7$ und $R^8$ obige Bedeutung besitzen und $R^{62}$ Nitro oder niederes Dialkylamino bedeutet,

mit einem den Rest $R^1$ liefernden Alkylierungsmittel behandelt, oder

g) ein 3,3-Dialkylbenzodiazepin-Derivat der allgemeinen Formel

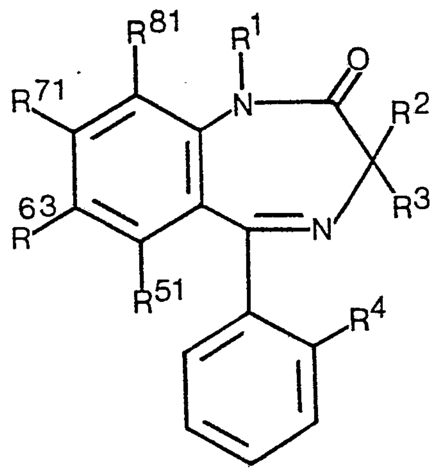

Ib

worin $R^1$, $R^2$, $R^3$ und $R^4$ obige Bedeutung besitzen, und entweder $R^{63}$ Nitro und $R^{51}$, $R^{71}$ und $R^{81}$ alle Wasserstoff bedeuten oder $R^{63}$ Amino oder niederes Alkylamino und $R^{81}$ Wasserstoff oder Halogen bedeuten, und einer der Reste $R^{51}$ und $R^{71}$ Wasserstoff und der andere Wasserstoff oder Halogen bedeutet,

halogeniert, oder

h) in einem 3,3-Dialkylbenzodiazepin-Derivat der obigen allgemeinen Formel Ia die primäre Aminogruppe in die Nitrogruppe überführt, oder

i) in einem 3,3-Dialkylbenzodiazepin-Derivat der allgemeinen Formel

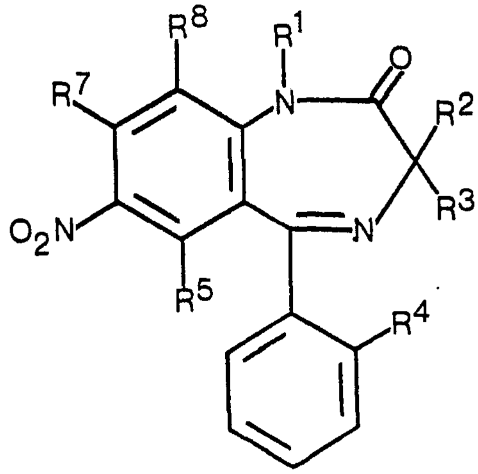

Ic

worin $R^1$, $R^2$, $R^3$, $R^4$, $R^5$, $R^7$ und $R^8$ obige Bedeutung besitzen,

die Nitrogruppe zur Aminogruppe reduziert, oder

j) in einem 3,3-Dialkylbenzodiazepin-Derivat der obigen allgemeinen Formel Ia die primäre Aminogruppe mono- oder dialkyliert, oder

k) in einem Dialkylbenzodiazepin-Derivat der allgemeinen Formel

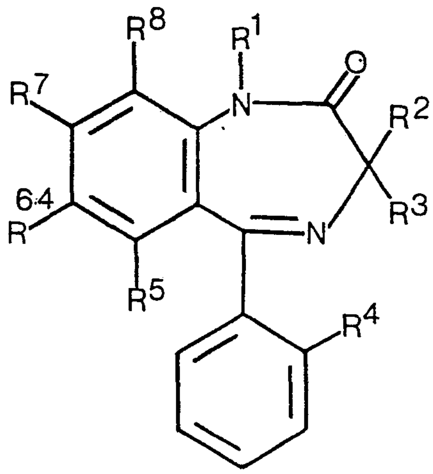

Id

worin $R^1$, $R^2$, $R^3$, $R^4$, $R^5$, $R^7$ und $R^8$ obige Bedeutung besitzen und $R^{64}$ niederes Alkylamino bedeutet,

die sekundäre Aminogruppe alkyliert, oder

l) ein 3,3-Dialkylbenzodiazepin-Derivat der obigen allgemeinen Formel Ia mit einem Dihalogenid der allgemeinen Formel

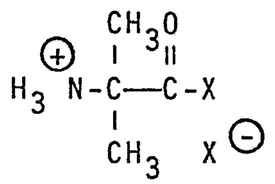 IX

worin X obige Bedeutung besitzt,

umsetzt, oder

m) ein 3,3-Dialkylbenzodiazepin-Derivat der allgemeinen Formel

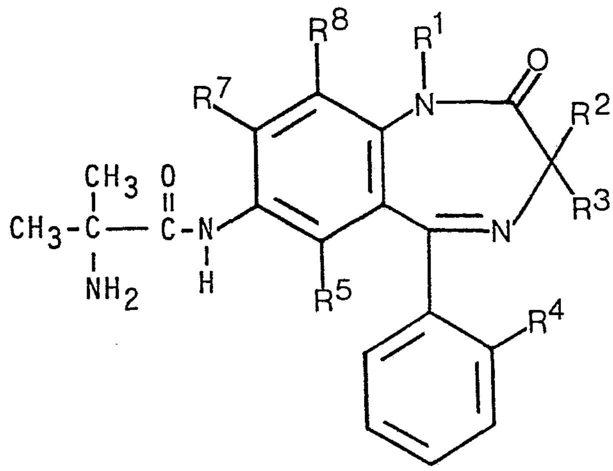 Ie

worin $R^1$, $R^2$, $R^3$, $R^4$, $R^5$, $R^7$ und $R^8$ obige Bedeutung besitzen,

mit Phosgen behandelt, oder

n) in einem 3,3-Dialkylbenzodiazepin-Derivat der allgemeinen Formel

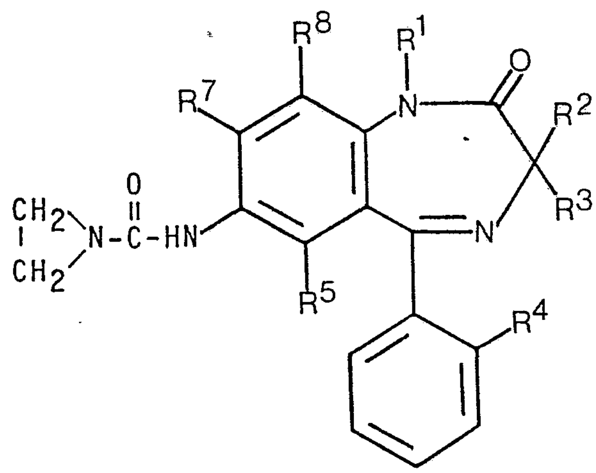 If

worin $R^1$, $R^2$, $R^3$, $R^4$, $R^5$, $R^7$ und $R^8$ obige Bedeutung besitzen,

den Aziridinring hydrolytisch öffnet, oder

o) ein 3,3-Dialkylbenzodiazepin-Derivat der allgemeinen Formel I in ein pharmazeutisch annehmbares Säureadditionssalz überführt.

2. Verfahren gemäss Anspruch 1, dadurch gekennzeichnet, dass man eine Verbindung der in Anspruch 1 definierten allgemeinen Formel I herstellt, worin $R^1$ Methyl bedëutet.

3. Verfahren gemäss Anspruch 1 oder 2, dadurch gekennzeichnet, dass man eine Verbindung der in Anspruch 1 definierten allgemeinen Formel I herstellt, worin $R^2$ und $R^3$ beide Methyl bedeuten.

4. Verfahren gemäss einem der Ansprüche 1 bis 3, dadurch gekennzeichnet, dass man eine Verbindung der in Anspruch 1 definierten allgemeinen Formel I herstellt, worin $R^4$ Fluor oder Chlor bedeutet.

5. Verfahren gemäss einem der Ansprüche 1 bis 4, dadurch gekennzeichnet, dass man eine Verbindung der in Anspruch 1 definienrten allgemeinen Formel I herstellt, worin $R^5$ Wasserstoff, Chlor oder Brom bedeutet.

6. Verfahren gemäss einem der Ansprüche 1 bis 5, dadurch gekennzeichnet, dass man eine Verbindung der in Anspruch 1 definierten allgemeinen Formel I herstellt, worin $R^7$ Wasserstoff, Chlor oder Brom bedeutet.

7. Verfahren gemäss einem der Ansprüche 1 bis 6, dadurch gekennzeichnet, dass man eine Verbindung der in Anspruch 1 definienrten allgemeinen Formel I herstellt, worin $R^8$ Wasserstoff oder Chlor bedeutet.

8. Verfahren gemäss einem der Ansprüche 1 bis 7, dadurch gekennzeichnet, dass man 7-Amino-9-chlor-5-(o-fluorphenyl)-1,3-dihydro-1,3,3-trimethyl-2H-1,4-benzodiazepin-2-on herstellt.

9. Verfahren gemäss einem der Ansprüche 1 bis 7, dadurch gekennzeichnet, dass man 7-Amino-6,8-dibrom-5-(o-fluorphenyl)-1,3-dihydro-1,3,3-trimethyl-2H-1,4-benzodiazepin-2-on herstellt.

10. Verfahren gemäss einem der Ansprüche 1 bis 7, dadurch gekennzeichnet, dass man 7-Amino-6,8-dibrom-5-(o-chlorphenyl)-1,3-dihydro-1,3,3-trimethyl-2H-1,4-benzodiazepin-2-on herstellt.

11. Verfahren gemäss einem der Ansprüche 1 bis 7, dadurch gekennzeichnet, dass man 7-Amino-6,8-dichlor-5-(o-chlorphenyl)-1,3-dihydro-1,3,3-trimethyl-2H-1,4-benzodiazepin-2-on herstellt.